(19) Europäisches Patentamt
European Patent Office
Office européen des brevets

(11) Numéro de publication : **0 644 264 A1**

(12) # DEMANDE DE BREVET EUROPEEN

(21) Numéro de dépôt : **94401855.5**

(22) Date de dépôt : **12.08.94**

(51) Int. Cl.$^6$ : **C12N 15/56,** C12N 15/60, C12N 15/70, C12N 9/24, C12N 9/88, C12N 1/21, // (C12N1/21, C12R1:19)

Une requête en rectification, page 33, lignes 31 et 32, a été présentée conformément à la règle 88 CBE. Il est statué sur cette requête au cours de la procédure engagée devant la division d'examen (Directives relatives à l'examen pratiqué à l'OEB, A-V, 3.).

(30) Priorité : **17.08.93 FR 9310050**

(43) Date de publication de la demande : **22.03.95 Bulletin 95/12**

(84) Etats contractants désignés : **AT BE CH DE DK ES FR GB GR IE IT LI LU NL PT SE**

(71) Demandeur : **SANOFI**
**32-34, rue Marbeuf**
**F-75008 Paris (FR)**

(72) Inventeur : **Legoux, Richard**
**F-31460 Le Saget (FR)**
Inventeur : **Lelong, Philippe**
**Les Ormes - Bât. A1**
**F-31320 Castanet Tolosan (FR)**
Inventeur : **Salome, Marc Louis Victor**
**Les Ormes II - Bât. F1**
**F-31320 Castanet Tolosan (FR)**

(74) Mandataire : **Le Guen, Gérard et al**
**CABINET LAVOIX**
**2, place d'Estienne d'Orves**
**F-75441 Paris Cédex 09 (FR)**
**Déclaration conformément à la règle 28(4) CBE(solution de l'expert)**

(54) **Enzyme recombiné destiné à la fragmentation du N-acétylhéparosane, gène codant pour l'enzyme et son utilisation.**

(57) L'invention concerne une séquence d'ADN qui porte le gène codant pour l'enzyme de fragmentation du N-acétylhéparosane et les séquences adjacentes permettant son expression. L'invention concerne aussi une enzyme destinée à la fragmentation du N-acétylhéparosane issue de ce gène et les procédés de fragmentation du N-acétylhéparosane de haute masse moléculaire utilisant cette enzyme.

EP 0 644 264 A1

La présente invention concerne un fragment d'ADN portant le gène codant pour l'enzyme de fragmentation du N-acétylhéparosane de haute masse moléculaire et les séquences adjacentes permettant l'expression de ce gène, une enzyme recombinée destinée à la fragmentation du N-acétylhéparosane contenant ce gène, l'obtention des préparations contenant cette enzyme recombinée ainsi que des procédés de fragmentation du N-acétylhéparosane utilisant cette enzyme.

Il est connu que certaines bactéries de l'espèce Escherichia coli, produisent un polysaccharide capsulaire, habituellement appelé K5, qui est une famille de polymères constituée d'unités répétées β-D-glucuronyl-1,4-α-N-acétyl-D-glucosaminyl (1,4), (W.F. Vann et al, Eur. J. Biochem, 1981, 116, 359-364), de structure (a) :

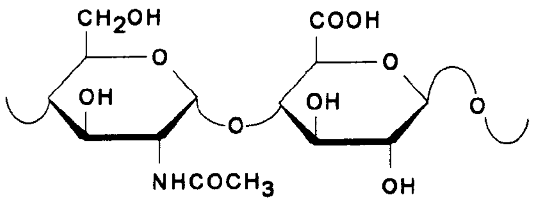

(a)

Ce polysaccharide sera appelé ici "N-acétylhéparosane". Ce produit possède au niveau des unités "acide uronique", une structure régulière composée uniquement d'acide D-glucuronique. Sa masse moléculaire est comprise entre $10^5$ et $2.10^6$ Da. Il s'agit donc d'un N-acétyhéparosane de haute masse moléculaire.

Le N-acétylhéparosane est utile comme matière première pour l'industrie pharmaceutique mais, pour cette utilisation, il a une masse moléculaire trop élevée. A titre d'exemple, on peut se référer aux demandes de brevets EP-0 333 243 et EP-0 489 647 qui concernent des dérivés de N-acétylhéparosane de petite masse moléculaire ou des produits obtenus à partir de N-acétylhéparosane de petite masse moléculaire. Les différents produits décrits dans ces deux demandes sont doués de propriétés pharmacologiques très intéressantes. Leurs masses moléculaires sont inférieures ou égales à 15000 Da.

Il est connu que le N-acétylhéparosane (polysaccharide K5) peut être fragmenté par une lyase phagique provenant d'un phage spécifique de la souche Escherichia coli (K5), mais cette fragmentation est très poussée et conduit à une disparition importante des fragments de masse moléculaire de 5000 Da vers des chaînes beaucoup plus petites, de masses moléculaires inférieures à 1000 Da (D. Gupta et al, FEMS Microbiology Letters, (1983), 16, 13-17). Ce type de fragmentation est utilisé dans la demande de brevet EP-A-0 333 243 pour la préparation de fragments contenant au maximum 10 unités saccharidiques.

Il a aussi été constaté que les cultures d'Escherichia coli (K5), souche SEBR 3282, produisent dans certaines conditions environnementales, une enzyme qui fragmente le N-acétylhéparosane au cours de la culture en fermenteur. La souche Escherichia coli (K5) SEBR 3282 répond positivement au test de typage par le phage K5 spécifique, selon la méthode de B. Kaiser et al (J. Clin. Microbiol., (1984), 19, 2, 264-266). Il s'agit donc bien d'une souche Escherichia coli (K5). Cette souche a été déposée auprès de la CNCM de l'Institut Pasteur, Paris, France, sous le N° I-1013.

L'enzyme obtenue à partir de la souche Escherichia coli (K5) SEBR 3282 a une masse moléculaire comprise entre 62000 et 70000 Da (mesurée par HPLC d'exclusion), son point isoélectrique se situe dans la zone de pH comprise entre 4,7 et 5,4 unités pH et elle est une éliminase.

Cette enzyme est aussi caractérisée en ce qu'elle agit de la façon suivante :

- elle est d'origine membranaire,
- la température de son fonctionnement optimal (activité maximale) est voisine de 37°C et la température à laquelle elle est inactivée est d'environ 60°C,
- la zone optimale de pH pour son fonctionnement se situe entre les valeurs pH 6 et pH 7,
- pour son fonctionnement, la zone optimale de concentration en ions monovalents ou divalents se situe au voisinage de 0,2 M.

L'enzyme obtenue à partir de la souche ci-dessus dénommée, est capable d'agir sur le N-acétylhéparosane de haute masse moléculaire et de le fragmenter en l'absence de corps bactérien in vitro et permet d'obtenir, dans certaines conditions, des fragments de N-acétylhéparosane se situant autour d'un pic de masse moléculaire d'environ 5000 Da, rassemblant à quelques unités disaccharidiques près, environ 70 % du produit.

Il a été constaté par ailleurs, qu'une préparation de cette enzyme convenablement solubilisée, permet d'obtenir des fragments de masse moléculaire plus élevée par rapport aux fragments obtenus spontanément, c'est à dire avec l'enzyme non solubilisée. En effet, des préparations d'enzyme solubilisée permettent de moduler la fragmentation et d'obtenir des fragments dont la majorité a une masse moléculaire supérieure d'au moins 1000 à 3000 Da par rapport à la masse moléculaire des fragments obtenus spontanément.

Toutefois, l'utilisation industrielle de cette enzyme et en particulier sous une forme soluble et des préparations la contenant est limitée par les faibles quantités disponibles à partir de la souche Escherichia coli (K5) SEBR 3282.

Par ailleurs, l'obtention des préparations de l'enzyme solubilisée nécessite un certain nombre d'opérations telle que la solubilisation de l'enzyme à l'aide de détergents ou de bases minérales fortes. Ces manipulations augmentent aussi le coût de préparation des différents N-acétylhéparosanes de petite masse moléculaire utilisés comme matière première dans l'industrie pharmaceutique.

Pour pallier ces inconvénients, des recherches dans ce domaine ont été suivies et ont permis l'isolement d'un fragment d'ADN portant le gène codant pour une enzyme de fragmentation du N-acétylhéparosane de haute masse moléculaire. L'introduction dans un vecteur d'expression du fragment d'ADN portant la séquence codant pour l'enzyme a permis d'obtenir de grandes quantités d'une enzyme recombinée destinée à la fragmentation du N-acétylhéparosane de haute masse moléculaire.

D'une manière surprenante, il a été constaté qu'à partir de ce même fragment d'ADN il est possible d'obtenir l'enzyme recombinée sous deux formes différentes : la première forme de l'enzyme est associée au culot cellulaire (soit environ 90 % de l'activité enzymatique) et est dite membranaire à l'instar de l'enzyme obtenue à partir de la souche sauvage ; la deuxième forme de l'enzyme se retrouve spontanément dans le surnageant, en quantité notable et est dite soluble.

En effet, il a été constaté qu'une séquence nucléotidique du fragment d'ADN cloné permet d'obtenir des constructions exprimant spontanément un fort pourcentage de l'enzyme sous forme soluble, c'est à dire directement dans le surnageant de culture. La forme soluble de l'enzyme préparée à partir du surnageant de culture et utilisée dans des conditions définies, permet de fragmenter le N-acétylhéparosane de haute masse moléculaire de manière complète et précise en fragments regroupés autour d'un pic majoritaire correspondant à 9-10 unités disaccharidiques, ce qui correspond à une masse moléculaire d'environ 5000 Da évaluée par HPLC d'exclusion. La dispersion des fragments autour de ce pic majoritaire est très faible. Une unité disaccharidique de N-acétylhéparosane correspond à l'unité disaccharidique représentée par la formule (a).

Cette possibilité, d'obtenir des fragments de N-acétylhéparosane faiblement dispersés au niveau de leur masse moléculaire, offerte par l'enzyme recombinée sous une forme soluble, dispense de tout fractionnement ultérieur par quelque méthode que ce soit, notamment par chromatographie d'exclusion.

De plus, en modifiant les conditions opératoires il est possible, toujours en faisant appel à la forme soluble de l'enzyme, d'obtenir des fragments de N-acétylhéparosane de taille inférieure ou supérieure aux 9-10 unités disaccharidiques répétitives citées ci-dessus. Pour obtenir des fragments dont la taille est inférieure aux 9-10 unités répétitives, il est aussi possible d'utiliser en particulier, les formes membranaires de l'enzyme.

Par ailleurs, quelque soit la forme, soluble ou membranaire de l'enzyme, la réaction enzymatique peut être conduite de manière incomplète pour obtenir un mélange de fragments de tailles supérieures aux 9-10 unités disaccharidiques citées ci-dessus.

La présente invention concerne donc un fragment d'ADN portant un gène codant pour l'enzyme destinée à la fragmentation du N-acétylhéparosane de haute masse moléculaire, l'enzyme recombinée codée par ce gène, les différentes formes de l'enzyme recombinée dérivée de cette séquence, notamment la forme membranaire et la forme soluble de cette enzyme, ainsi que des procédés de fragmentation utilisant cette enzyme recombinée.

Le fragment d'ADN portant le gène codant pour l'enzyme destinée à la fragmentation du N-acétylhéparosane et les systèmes d'expression permettant sa biosynthèse, est caractérisé en ce qu'il correspond à la séquence nucléotidique SEQ IDN° 1.

Ce fragment d'ADN, dont la taille exacte est 3089 paires de bases (pb) porte le gène codant pour l'enzyme de fragmentation du N-acétylhéparosane de haute masse moléculaire, mais également les signaux de régulation permettant de l'exprimer. Ces signaux de régulation sont principalement :

1- Un promoteur spécifique, essentiel pour la transcription du gène

2- Un site de fixation des ribosomes, essentiel pour effectuer la traduction de la protéine à partir du ARNm transcrit.

L'analyse informatique fait apparaître plusieurs phases de lecture. Par phase de lecture on entend une séquence d'acides aminés déduite de la séquence nucléotidique par l'intermédiaire du code génétique qui commence par une méthionine codée par ATG et qui finit par l'un des codons stop TAA, TGA ou TAG.

Il existe dans ce fragment d'ADN, plusieurs phases de lecture possibles dont la taille est inférieure à 100 acides aminés. Il existe également une phase de lecture de 820 acides aminés caractérisée en ce qu'elle a la séquence peptidique SEQ ID N° 2.

La séquence SEQ ID N° 2 est aussi l'objet de la présente invention.

L'invention concerne aussi tout fragment d'ADN comportant la phase de lecture ou partie de celle-ci donnant la séquence peptidique SEQ ID N° 2 ou issu de la séquence peptidique SEQ ID N° 2.

L'invention concerne aussi une souche portant un plasmide permettant la production d'une enzyme recombinée fragmentant le N-acétylhéparosane de haute masse moléculaire. Une telle souche a été déposée auprès de la CNCM de l'Institut Pasteur, Paris, France, le 6 août 1993, sous le numéro I-1352.

La souche portant un plasmide permettant la production d'une enzyme recombinée fragmentant le N-acétylhéparosane de haute masse moléculaire est une souche recombinée de la souche Escherichia coli RRI (K12) fournie par GIBCO-BRL (réf. 530-8231 SA). D'autres souches d'Escherichia coli appropriées peuvent être envisagées.

Comme vecteur il a été utilisé le vecteur pUC 18 hydrolysé par BamHI/BAP (Pharmacia® réf. 27-4855-01), dans lequel il a été introduit le fragment d'ADN correspondant à la séquence SEQ ID N° 1.

Toutefois pour obtenir une souche portant un plasmide permettant la production d'une enzyme recombinée fragmentant le N-acétylhéparosane de haute masse moléculaire, il est possible d'envisager l'utilisation d'autres vecteurs et/ou comme il a été indiqué précedemment, d'autres souches réceptrices.

Comme autres vecteurs, il est possible d'utiliser des vecteurs tels que le pBR 322 (Pharmacia® réf. 274902-01) ou tout autre vecteur dans lequel est insérable tout le fragment d'ADN correspondant à la séquence SEQ ID N° 1 ou partie de ce fragment d'ADN portant la séquence nécessaire à l'expression de la protéine de 820 acides, cette séquence traduite étant la séquence SEQ ID N° 2.

A part la souche réceptrice Escherichia coli RRI (K12), on peut utiliser d'autres souches Gram négatif à condition toutefois que le système de régulation de la protéine contenant 820 acides aminés et ayant la séquence peptidique SEQ ID N° 2 soit compatible avec la cellule hôte.

La souche déposée auprès de la CNCM de l'Institut Pasteur, Paris, France, le 6 août 1993, sous le numéro I-1352 est une souche obtenue par transformation de la souche Escherichia coli RRI (K12) avec un vecteur portant un fragment d'ADN correspondant à la séquence SEQ ID N° 1 et donc,portant la séquence nécessaire à l'expression de la protéine de 820 acides aminés qui est la séquence SEQ ID N° 2 (séquence traduite).

Toutefois il est possible d'envisager un certain nombre de souches productrices d'enzyme recombinée destinée à la fragmentation du N-acétylhéparosane de haute masse moléculaire obtenue par transformation de la souche Escherichia coli RRI (K12), ou d'autres souches Gram négatif adéquates, avec des vecteurs portant des fragments d'ADN issus de la séquence SEQ ID N° 1 et portant au maximum les séquences nécessaires à l'expression de la protéine de 820 acides aminés. Ces séquences traduites sont :

- soit la séquence SEQ ID N° 2 en sa totalité, correspondant aux acides aminés 1 à 820,
- soit l'ensemble des acides aminés 112 à 820 de la séquence SEQ ID N°2,
- soit l'ensemble des acides aminés 160 à 820 de la séquence SEQ ID N° 2,
- soit l'ensemble des acides aminés 194 à 820 de la séquence SEQ ID N° 2.

L'invention concerne donc en particulier un fragment d'ADN codant pour tout ou partie de la séquence SEQ ID N° 2, comme indiqué ci-dessus.

L'invention concerne notamment un gène codant pour une enzyme destinée à la fragmentation du N-acétylhéparosane porté par le fragment d'ADN de la séquence nucléotidique SEQ ID N° 1.

L'invention concerne plus particulièrement une enzyme recombinée destinée à la fragmentation du N-acétylhéparosane de haute masse moléculaire issue du gène ci-dessus mentionné. Ce gène comporte une séquence d'ADN codant pour tout ou partie de la séquence peptidique SEQ ID N° 2.

Des vecteurs d'expression caractérisés en ce qu'ils portent avec les moyens nécessaires à l'expression, le fragment d'ADN codant pour la protéine SEQ ID N° 2 capable de fragmenter la N-acétylhéparosane font aussi partie de l'invention.

Pour obtenir l'enzyme recombinée objet de la présente invention on a utilisé comme vecteur le vecteur p466 qui est décrit de manière détaillée dans la demande de brevet EP- 0 480 461.

Toutefois, il est aussi possible d'utiliser d'autres vecteurs d'expression, par exemple ceux décrit par Studier FW et Moffatt BA dans J. Mol. Biol (1986), 189, pp.113-130 et de transformer une des souches réceptrices préconisées par ces auteurs.

L'invention a aussi plus particulièrement comme objet le plasmide p868,26. Ce plasmide a été obtenu en liguaturant un fragment du plasmide p466 avec un fragment d'ADN obtenu après la réaction de polymérase en chaîne en utilisant un oligonucléotide dont la séquence est la suivante :

5'-GAT-CCA-TAT-GAC-GGT-CTC-AAC-CGA-AGT-TG-3'

Région 1 Région 2

SEQ ID N° 3

en présence d'un oligonucléotide dont la séquence est la suivante :

5'-GAT-CAA-GCT-TAT-CAA-TTC-CCT-GTT-AAT-TGC-AAA-AC-3'

Région 1 Région 2

SEQ ID N° 7

A = acide désoxyadénosine-5'-phosphorique
C = acide désoxyguanosine-5'-phosphorique
G = acide désoxycytidine-5'-phosphorique
T = acide désoxythymidine-5'-phosphorique

L'invention a aussi pour objet une souche bactérienne, la souche bactérienne TP 2339 transformée par le plasmide p868,26. Cette souche permet d'obtenir l'enzyme recombinée objet de la présente invention à la fois sous forme soluble et sous forme membranaire.

Chez la souche Escherichia coli sauvage, l'induction de l'enzyme est dépendante de manière complexe des composants du milieu de culture. A l'opposé, le mode d'obtention de l'enzyme recombinée, objet de la présente invention, permet de s'affranchir de matières premières naturelles complexes en particulier d'origine animale. En effet, le gène codant pour l'enzyme recombinée est placé sous le contrôle d'un promoteur inductif rendu fonctionnel par simple addition d'IPTG (isopropyl-β-D-thiogalactoside) au moment jugé le plus approprié pour le but recherché. Par exemple, le moment choisi pour l'induction sera situé en phase exponentielle de la culture, à la moitié de la biomasse maximale prévisible.

Les techniques utilisées pour l'introduction d'un fragment d'ADN codant pour une enzyme de fragmentation du N-acétylhéparosane de haute masse moléculaire dans un vecteur d'expression permettant d'obtenir de grandes quantités de cette enzyme, sont des techniques bien connues de l'homme de l'art. Elles sont exposées en détail dans l'ouvrage de Sambrook Fritsch et Maniatis "Molecular cloning : A Laboratory manual" publié en 1989 par les édition Cold Spring Harbor Press (N.Y.) 2ème édition.

L'enzyme recombinée objet de la présente invention permet de fragmenter le N-acétylhéparosane et d'obtenir des fragments comportant à l'extrémité non réductrice un reste d'acide glucuronique ayant une double liaison entre les carbones 4 et 5 (élimination du groupe OH). De telles enzymes ne font pas intervenir l'eau dans la réaction chimique considérée et sont dites de type éliminase. L'enzyme recombinée, objet de la présente invention est donc une éliminase et notamment une endo-β-éliminase.

L'enzyme recombinée, objet de la présente invention peut être utilisée pour la fragmentation du N-acétylhéparosane de haute masse moléculaire soit sous sa forme membranaire soit sous sa forme soluble.

La forme membranaire de l'enzyme recombinée se retrouve spontanément dans le culot bactérien après centrifugation ou filtration de la culture de la souche recombinée. Une extraction du culot en présence de Triton X-100 permet d'obtenir l'enzyme recombinée sous forme soluble. Cette extraction est faite par exemple en utilisant un tampon d'extraction constitué de :

Tris.HCl 25 mM
NaCl 9 g/l
Triton X-100 30 g/l
(pH=7,0)

Le culot bactérien de la souche Escherichia coli enzyme recombinée est resuspendu dans le tampon d'extraction. Après 1 heure d'attente à température ambiante, la suspension de culture est centrifugée et le surnageant conservé. Le surnageant est utilisable tel que car le Triton X-100 ne présente pas d'effet inhibiteur sur l'enzyme.

La forme soluble de l'enzyme se retrouve aussi spontanément dans le surnageant de la culture de la souche recombinée. On peut donc l'isoler dans le surnageant en éliminant les bactéries de la culture par centrifugation ou par microfiltration.

L'enzyme recombinée soluble peut-être conservée à la température de -20°C.

Le terme "préparation contenant l'enzyme" inclut aussi bien les préparations contenant la forme membranaire de l'enzyme que les préparations contenant la forme soluble de l'enzyme.

Les préparations contenant l'enzyme recombinée sous forme soluble ou sous forme membranaire font aussi partie de l'invention. Les différentes préparations contenant l'enzyme recombinée objet de la présente invention et notamment les préparations contenant l'enzyme sous forme membranaire ou sous forme soluble sont particulièrement utiles pour préparer des N-acétylhéparosanes de petite masse moléculaire, pour les raisons suivantes :

- le N-acétylhéparosane de haute masse moléculaire s'obtient sur milieu synthétique peu coûteux avec des rendements supérieurs à ceux d'un milieu complexe, milieu nécessaire à l'obtention d'un N-acétylhéparosane de petite masse moléculaire ;
- le N-acétylhéparosane de haute masse moléculaire est techniquement plus facile à manipuler que celui de petite masse moléculaire ;
- la fragmentation in vitro sépare la phase de production de celle de fragmentation, ce qui permet de maîtriser et d'optimiser les deux phases tout en offrant une grande latitude sur les caractéristiques du produit recherché, par exemple sur sa masse moléculaire ;
- l'enzyme recombinée et le N-acétylhéparosane de haute masse moléculaire sont particulièrement stables, ce qui autorise des recyclages multiples éventuellement nécessaires dans la mise en oeuvre de procédés dynamiques, au cours desquels les processus de fragmentation et de fractionnement sont mis en oeuvre simultanément ou successivement tout en s'affranchissant des contraintes de temps liées à l'emploi d'un bioréacteur ;
- une souche recombinée, contrairement à la souche sauvage Escherichia coli (K5) SEBR 3282, permet de fragmenter de grandes quantités de N-acétylhéparosane de haute masse moléculaire. En effet, 1 litre de surnageant de culture contenant l'enzyme recombinée est capable de fragmenter environ 10-100 litres de culture de N-acétylhéparosane, ce qui correspond à 30-400 g de N-acétylhéparosane de haute masse moléculaire.

Pour obtenir un N-acétylhéparosane de petite masse moléculaire ayant des fragments correspondant à 9-10 unités disaccharidiques, on utilise de préférence l'enzyme recombinée soluble obtenue spontanément dans le surnageant de culture de la souche recombinée objet de la présente invention. La concentration finale du N-acétylhéparosane de haute masse moléculaire soumis à l'action de l'enzyme soluble est de 2-30 g/l, plus particulièrement de 8-12 g/l, de préférence 10 g/l.

L'enzyme soluble est diluée de telle sorte, qu'une quantité de N-acétylhéparosane de haute masse moléculaire supérieure de 10-100 % de préférence 50 % à celle effectivement utilisée qui est de 10 g/l puisse être fragmentée au cours d'une réaction durant de 2 heures à 48 heures de préférence de 16 à 24 heures.

La réaction enzymatique est effectuée de préférence en présence de chlorure de sodium à pH 7 et à 37°C sous agitation douce. La réaction est suffisamment lente pour permettre un suivi de la fragmentation du N-acétylhéparosane de haute masse moléculaire. Ce suivi est effectué par l'évaluation de masse moléculaire du N-acétylhéparosane fragmenté par HPLC d'exclusion.

Pour obtenir des fragments de N-acétylhéparosane de taille inférieure à 9-10 unités disaccharidiques, plusieurs alternatives sont possibles et notamment :
- soit l'enzyme soluble est utilisée en présence d'ions calcium en concentration notable,
- soit on utilise l'enzyme recombinée sous forme membranaire,
- soit la durée de la réaction de la fragmentation enzymatique est augmentée,
- soit la concentration de l'enzyme diluée utilisée pour la fragmentation du N-acétylhéparosane de haute masse moléculaire est augmentée.

Comme sel de calcium, on peut utiliser le chlorure de calcium. La concentration en ions calcium est de l'ordre de 1-300 mM, de préférence de 100mM.

Dans le but d'obtenir des fragments de N-acétylhéparosane de taille supérieure à 9-10 unités disaccharidiques, il suffit de procéder à une réaction enzymatique partielle, par exemple en limitant sa durée. Dans ce cas, les différentes formes de l'enzyme recombinée (membranaire ou soluble) peuvent être employées indifféremment.

Pour arrêter la réaction enzymatique, on peut utiliser plusieurs moyens et notamment :
- le froid puisqu'à 4°C, l'activité de l'enzyme recombinée est ralentie,
- la chaleur puisque le N-acétylhéparosane résiste à une température d'environ 60°C et que l'enzyme est inactivée à cette température,
- le pH basique ; l'enzyme recombinée et le N-acétylhéparosane résistent à pH 11 mais l'activité de l'enzyme dans cette zone de pH est ralentie ou nulle,
- le pH acide, par exemple pH 4 ou,
- l'éthanol à 80 % qui inactive l'enzyme et précipite le N-acétylhéparosane.

L'invention concerne aussi des procédés de fragmentations du N-acétylhéparosane de haute masse moléculaire mettant en oeuvre l'enzyme recombinée, objet de la présente invention.

Pour la réalisation de l'invention la préparation de N-acétylhéparosane majoritairement de haute masse moléculaire s'est avérée nécessaire. Elle ne fait pas partie de l'invention mais cette préparation est aussi exposée de manière détaillée ci-dessous.

L'obtention du fragment d'ADN portant le gène codant pour l'enzyme de fragmentation du N-acétylhéparosane et les séquences adjacentes permettant son expression, sa caractérisation ainsi que les différents outils de génie génétique et procédés qui ont permis l'obtention de l'enzyme recombinée, sont décrits de manière détaillée ci-après dans les différentes "Sections".

Un exemple de la mise en oeuvre de l'enzyme pour la fragmentation du N-acétylhéparosane de haute masse moléculaire illustre l'utilisation de l'enzyme recombinée objet de la présente invention.

Il est donné à titre non-limitatif.

## PREPARATION

## N-ACETYLHEPAROSANE MAJORITAIREMENT DE HAUTE MASSE MOLECULAIRE

On ensemence 400 ml du milieu B, de composition précisée dans le Tableau I ci-après, avec la souche Escherichia coli (K5) SEBR 3282 déposée auprès de la CNCM de l'Institut Pasteur, Paris - France, sous le N° I-1013, et on incube sous agitation pendant 2 heures à 37°C. La préculture obtenue est alors transférée dans un fermenteur de 18,5 litres contenant 11 litres du milieu A, de composition précisée aussi dans le Tableau I ci-après, et on incube pendant 6 heures et 30 minutes à 37°C et pH égal à 7,2, la pression partielle en oxygène étant maintenue à 40 mmHg par régulation de l'injection d'air (jusqu'à 20 l/minute) et de l'agitation. On ajoute alors du glycérol en introduisant de façon continue une solution stérile contenant 500 g/l de glycérol à raison de 18 g/h pendant 16-17 heures.

On poursuit la culture dans les mêmes conditions de température, de pH et de pression partielle en oxygène jusqu'à consommation quasi totale du glycérol. Le suivi de la densité optique (DO) à $\lambda$ = 600 nm de la suspension de la culture après la fin de l'ajout en glycérol, montre un état stationnaire ou de lyse légère jusqu'à l'arrêt de la culture à 28-30 heures d'âge en fermenteur.

On refroidit alors la suspension de culture à 25°C qui est centrifugée de 11000 à 14000 g pendant 15 à 20 minutes. Le surnageant est additionné de 0,1 M NaOH (le pH monte à environ 10) et centrifugé à nouveau. Le surnageant résultant est alors concentré sans attendre sur cartouches à fibres creuses Amicon® de seuil de coupure 10 kD ou équivalent puis la solution est neutralisée (pH = 7). On obtient ainsi une solution enrichie en N-acétylhéparosane de haute masse moléculaire. On ajoute à la solution une quantité adéquate de chlorure de sodium pour avoir une concentration finale de 0,5 M en NaCl, puis on ajoute 4 volumes d'éthanol. On laisse se former le précipité pendant une nuit à 4°C. Après une première élimination du surnageant par pompage, le précipité est centrifugé à 5000 g pendant 20 minutes à température ambiante. On reprend les culots de centrifugation dans l'éthanol, on agite la suspension obtenue, et on la laisse reposer pendant 1 heure à température ambiante. On répète les opérations de centrifugation et de mise en suspension. On centrifuge à nouveau à 5000 g pendant 20 minutes. On sèche les culots de centrifugation obtenus dans une étuve sous vide à 40°C pendant 24 heures. Le N-acétylhéparosane ainsi obtenu est un "N-acétylhéparosane de haute masse moléculaire purifié".

## TABLEAU I

**Composition et préparation du milieu A et du milieu B.**

**MILIEU A**

Dans 900 ml d'eau ultra-purifiée dissoudre dans l'ordre :

| | | |
|---|---|---|
| Tricine ® N'[Tris-(hydroxyméthyl) méthyl] glycine)* | 360 | mg |
| $K_2HPO_4$ | 790 | mg |
| Acide glutamique | 11000 | mg |
| $MgCl_2$, $6H_2O$ | 500 | mg |
| $K_2SO_4$ | 450 | mg |
| $FeSO_4$, $7H_2O$ | 18 | mg |
| $CaCl_2$, $2H_2O$ | 2 | mg |
| NaCl | 500 | mg |
| KCl | 5000 | mg |
| Solution d'oligo-éléments (cf. Tableau II) | 1 | ml |
| Glycérol | 10000 | mg |

Ajuster le pH à 7,2 avec de la potasse concentrée de densité 1,38 et compléter à 1000 ml avec de l'eau ultra-purifiée. Effectuer une filtration stérilisante sur membrane de 0,2 µm.

Solution de glycérol

Dissoudre 50 g de glycérol dans une quantité adéquate d'eau ultra-purifiée et ajuster le volume à 1000 ml avec le même solvant. Effectuer une filtration stérilisante sur membrane de 0,2 µm.

L'anti-mousse employé en cours de fermentation est le Struktol J 673® (Schill et Seilacher).

**MILIEU B**

La préparation du milieu B est identique à celle du milieu A, à la différence près, qu'il convient d'ajouter en plus le tampon (pH 7,2) : acide 3-morpholinopropanesulfonique (M.O.P.S.) après addition de l'agent anti-mousse.

* (commercialisé par Fluka)

## TABLEAU II

| Préparation de la solution d'oligo-éléments | | |
|---|---|---|
| dans 800 ml d'eau ultra-purifiée dissoudre (dans l'ordre) : | | |
| $H_3BO_3$ | 500 | mg |
| $Na_2MoO_4,2H_2O$ | 1930 | mg |
| $CoCl_2,6H_2O$ | 11850 | mg |
| $CuSO_4,5H_2O$ | 25 | mg |
| $ZnSO_4,7H_2O$ | 2000 | mg |
| $AlCl_3,6H_2O$ | 2410 | mg |
| Ajouter 100 ml d'acide chlorhydrique de densité 1,19 et compléter à 1000 ml avec de l'eau ultra-purifiée. | | |

## SECTIONS

### 1 - PREPARATION DE L'ADN GENOMIQUE DE LA SOUCHE D'ESCHERICHIA COLI SEBR 3282

Une colonie de la souche Escherichia coli (K5) SEBR 3282 est mise en culture à 37°C sous agitation dans 5 ml de milieu LB (Bactotryptone 5 g/l, Extrait de levure 10 g/l, NaCl 5 g/l : pH 7,35) pendant une nuit. Un millilitre de cette culture est utilisé pour ensemencer une fiole de 1 litre contenant 100 ml du même milieu de culture et incubé à 37°C sur un agitateur orbital réglé à 180 rotations par minute. Lorsque la densité optique de la culture mesurée à $\lambda$ = 600 nm est égale à 1, les bactéries sont centrifugées quelques minutes à 10000 tr/min. (Beckman J2.21) et reprises dans 20 ml d'un tampon de composition suivante : Tris-HCl pH 8,0, 10 mM, EDTA 20 mM, SDS 0,1 %, Protéinase K (Sigma® P 2308) 50 µg/ml

Cette suspension bactérienne est incubée 1 heure à 60°C. La réaction enzymatique est arrêtée par deux extractions au phénol (Appligène® réf. 130181) suivie d'une extraction au dichlorométhane. Les acides nucléiques sont précipités en présence de NaCl 0,1 M et de deux volumes d'éthanol, et resolubilisés dans 10 ml d'un tampon TE (Tris HCl, pH 8,0, 10 mM; EDTA 1 mM) contenant 10 µg/ml de RNase (Boehringer Mannheim® 1119915)

Après une heure d'incubation à 37°C, la solution est de nouveau soumise à deux extractions au phénol suivies d'une extraction au dichlorométhane. L'ADN obtenu est précipité en présence de 0,1 M de NaCl et de deux volumes d'éthanol puis resolubilisé par 10 ml de tampon TE. Une fraction aliquote de cet ADN, environ 10 µg, est soumis à une hydrolyse partielle par l'enzyme de restriction SAU3A (Appligène ®) dans les conditions recommandées par le fournisseur.

L'ADN partiellement hydrolysé est soumis à une électrophorèse sur gel d'agarose 1 % en présence de la référence de taille 1 Kpb (kilo paires de bases) (BRL réf. 520-5615 SA). La bande d'ADN qui correspond à une taille d'environ 3 Kpb, est purifiée par la technique du geneclean (Bio 101® réf. 3105).

Une fraction aliquote (approximativement 200 ng) de la bande d'ADN génomique, hydrolysée par SAU3A et dont la taille est d'environ 3 Kpb, est ligaturée selon la technique décrite dans "Molecular cloning : A Laboratory manual" (Sambrook, Fritsch et Maniatis, Cold Spring Harbor Press (N.Y.) 2ème édition (1989) pp. 1:63-1:67) en présence du vecteur pUC 18 hydrolysé par BamHI et déphosphorylé (Pharmacia® Réf. 27-4855-01) dans le mélange réactionnel approprié décrit "Molecular cloning : A Laboratory manual" (document référencé ci-avant) pendant une nuit à 4°C.

Ce mélange de ligature est utilisé pour transformer, dans les conditions recommandées par le fournisseur, la souche Escherichia coli RRI (K12) (GIBCO-BRL réf. 530-8261 SA). Il en résulte un mélange appelé "mélange de transformation" qui est étalé après dilution sur 10 boîtes de Pétri contenant le milieu LB précédemment décrit et supplémenté avec 15 g/l d'agar noble et 100 mg/ml d'ampicilline.

Après une nuit d'incubation à 37°C, les boîtes de Pétri se recouvrent d'environ 700 colonies issue chacune d'un clone. Chaque boîte de Pétri correspond à un pool et les dix pools sont classés de A à J et regroupés sous le numéro 838. Chaque clone possède un fragment d'ADN de taille comprise entre 3 et 4 Kpb. L'ensemble des dix pools représente approximativement 4 fois le génome entier de Escherichia coli.

## 2- PURIFICATION DU CLONE 838,7 PRODUCTEUR DE L'ENZYME DESTINEE A LA FRAGMENTATION DU N-ACETYLHEPAROSANE

Le clone présentant une activité de type éliminasique a été mis en évidence par la fragmentation de N-acétylhéparosane de haute masse moléculaire in vitro. La fragmentation de N-acétylhéparosane de haute masse moléculaire a été suivie par analyse de la masse moléculaire du N-acétylhéparosane fragmenté par HPLC d'exclusion.

### 2.1- Incubation des pools de bactéries transformées

Les 10 pools précédents constitués par le mélange de transformation décrit dans la Section 1, sont étalés en boîtes de Pétri sur milieu LB gélosé (avec agar) supplémenté de 100 µg/ml d'ampicilline et incubés 48 heures à 37°C.
Le tapis bactérien résultant, est resuspendu dans 3 ml de tampon A (de composition suivante: 100 mM bis-Tris-propane ; 150 mM NaCl, le pH est ajusté à 6,6 avec de l'acide chlorhydrique). Ces suspensions bactériennes sont utilisées pour effectuer la réaction enzymatique de fragmentation.

### 2.2- Réaction enzymatique de fragmentation

A 500 µl de tampon A décrit précédemment, sont ajoutés 50 µl d'une solution de N-acétylhéparosane de haute masse moléculaire à 20 g/l. Pour obtenir cette solution on dissout dans l'eau ultra purifiée une quantité adéquate de N-acétylhéparosane de haute masse moléculaire purifiée (Préparation). Le mélange réactionnel ainsi constitué est incubé de 3 à 4 jours dans un tube Eppendorf à 40°C.

### 2.3- Analyse des produits de fragmentation

Préparation des échantillons

Le mélange réactionnel en tube Eppendorf est alors centrifugé à 10000 g pendant 3 minutes. 100 µl du surnageant sont précipités par l'addition de 4 volumes d'éthanol puis à nouveau centrifugés dans les mêmes conditions. Le culot résultant est repris dans 300 µl de tampon pipérazine 25 mM, pH 3,5 (0,215 g de pipérazine anhydre sont dissous dans 90 ml d'eau ultra-purifiée ; le pH est ajusté à 3,5 avec HCl 1M puis le volume est ajusté à 100 ml avec de l'eau ultra-purifiée) et passé dans une microcolonne préremplie avec 100 µl de résine Q-Sépharose®. Le lavage de la microcolonne est effectué avec 2 ml de tampon pipérazine 25 mM (pH 3,5) suivi de 2 ml d'eau ultra-purifiée. L'élution se fait avec 200 µl de NaCl 0,5 M. L'éluat ainsi préparé est utilisé directement pour l'analyse en chromatographie HPLC d'exclusion.

Conditions opératoires en HPLC d'exclusion

Un échantillon de 65 µl de cet éluat contenant le N-acétylhéparosane purifié est analysé par chromatographie d'exclusion sur une chaîne HPLC isocratique. Les conditions opératoires sont les suivantes :

- Colonne : TSK 2000 SW® (Toso-Haas) de 300 x 7,5 mm constituée de billes de silice de 1 µm de diamètre et de 125 Å de porosité.
- Eluant : solution aqueuse de sulfate de sodium 0,5 M, filtrée sur filtre de 0,2 µm et dégazée
- Débit : 1 ml/min.
- Détection UV : 205 nm
- L'étalonnage est effectué à l'aide d'une gamme d'oligosaccharides dérivés de l'héparine (10 mg) de masses moléculaires suivantes (Da) : 3410, 4000, 4540, 5000, 6150, 7540, 10090.

Interprétation des résultats

Sur les graphes obtenus le tracé compris entre le sommet du pic correspondant aux espèces de haute masse moléculaire et le pic correspondant à la sortie du chlorure de sodium est examiné. Si cette ligne s'infléchit vers le haut par rapport au témoin, -ce qui traduit la présence de fragments de N-acétylhéparosane de plus petite taille-, ceci est considéré comme la signature d'une activité de fragmentation et donc de la présence de l'enzyme recherchée dans le pool à l'origine du mélange réactionnel.

### 2.4- Résultats

Le pool I, parmi les 10 pools notés de A à J, s'est révélé positif et a été utilisé par la suite. Un échantillon aliquote conservé en glycérol à - 80°C a été ré-étalé sur boîte, et 1200 clones ont été obtenus. Ces clones ont été analysés 10 par 10 et finalement un clone producteur appelé plasmide p838,7 a été retenu.

### 3- ANALYSE DU PLASMIDE P838,7

La souche d'Escherichia coli (K12) RRI transformée par le plasmide p838,7 est mise en culture dans 500 ml de milieu LB supplémenté avec 100 µg/ml d'ampicilline. Cette souche a été déposée auprès de la CNCM de l'Institut Pasteur, Paris, France le 6 août 1993, sous le numéro I-1352.
Le plasmide présent dans cette souche est purifié par la méthode du Qiagen® (Diagen® réf. 10.043). Le plasmide appelé p838,7 est hydrolysé suivant les techniques connues de l'homme de l'art par plusieurs enzymes de restriction : EcoRI, HindIII, PstI, SphI, XbaI, Sca1 (New England Biolabs Beverly MA 01915-5599 USA). L'analyse de la carte de restriction obtenue montre que la taille du fragment d'ADN provenant de l'ADN génomique de la souche d'Escherichia coli (K5) SEBR 3282 est d'environ 3100 pb. La détermination complète de la séquence est ensuite effectuée par la technique de Sanger décrite dans PNAS (1977), 74, pp. 5463-5467.
La séquence nucléotidique complète du fragment d'ADN portant le gène codant pour l'enzyme de fragmentation de N-acétylhéparosane de haute masse moléculaire et les systèmes d'expression permettant sa biosynthèse, est la séquence $S_1$.

### 4- ANALYSE DE LA SEQUENCE NUCLEOTIDIQUE DU FRAGMENT D'ADN PORTEUR DE L'ACTIVITE ENZYMATIQUE

Le fragment d'ADN de séquence $S_1$ dont la taille exacte est 3089 pb, porte le gène codant pour l'enzyme de fragmentation de N-acétylhéparosane de haute masse moléculaire mais également les signaux de régulation permettant de l'exprimer. En effet, ce fragment seul est capable d'assurer la production de l'enzyme de fragmentation du polysaccharide N-acétylhéparosane de haute masse moléculaire. Ces signaux de régulation sont en particulier :

a- un promoteur spécifique, essentiel pour la transcription du gène.

b- un site de fixation des ribosomes, essentiel pour effectuer la traduction de la protéine à partir du mRNA transcrit.

L'analyse informatique de la séquence nucléotidique fait apparaître plusieurs phases de lecture. Une phase de lecture est une séquence d'acides aminés déduite de la séquence nucléotidique, par l'intermédiaire du code génétique, qui commence par une méthionine codée par ATG, et qui finit par l'un des codons stop TAA, TGA ou TAG. (Sambrook, Fritsch et Maniatis, Cold Spring Harbor Press (N.Y.) 2ème édition (1989) pp. D1). Il existe dans la séquence $S_1$, plusieurs phases de lecture possibles dont la taille est inférieure à 100 acides aminés. Ces dernières ne seront pas décrites.

Il existe également une phase de lecture dont la taille est de 820 acides aminés. La figure 1 montre la séquence nucléotidique double brin du fragment $S_1$ sur laquelle est calquée la phase de lecture de 820 acides aminés.

La séquence de la phase de lecture la plus longue dont la taille est de 820 acides aminés est la séquence $S_2$. Cette séquence est comparée à celle des protéines contenues dans les banques de données.
Sur cette séquence on observe :

a- L'existence d'une homologie avec une enzyme de l'espèce Erwinia chrysanthemi possédant une activité exo-poly-α-D-galacturonidase. Cette enzyme est décrite dans J. Bacteriol. (1990), 172, pp. 4988-4995 et PNAS (1992), 83, pp. 8990-8994. Le premier document décrit sa séquence de manière détaillée.

b- La répétition de la séquence « asparagine (Asn)-A-B-sérine (Ser) », où A est isoleucine (Ile), leucine (Leu) ou tyrosine (Tyr) et B valine (Val), isoleucine (Ile) ou alanine (Ala), aux positions 384-387, 411-414, 433-436, 461-464, et 495-498. L'une de ses séquences « Asn-Ile-Ala-Ser » en position 461-484 fait partie d'un site potentiel d'attachement des lipoprotéines aux lipides. Cette séquence de fixation est connue dans la littérature (Hayashi S., Wu H.C. J. dans Bioenerg. Biomembr., (1990), 22, pp. 451-471 ; Klein P., Somorjai R.L. Lau P.C.K., Protein Eng., (1988), 2, pp. 15-20 ; von Heijne G. dans Protein Eng., (1989), 2, pp. 531-534 ; Mc Gavin M.J., Forsberg C.W., Crosby B., Bell A.W., Dignard D., Thomas D.Y. dans J. Bacteriol. (1989), 171, pp. 5587-5595 ; Rothe B., Roggentio P., Frank R., Bloecker H., Schauer R. dans J. Gen. Microbiol. (1989), 135, pp. 3087-3096).

D'après un programme informatique contenu dans "UWGCG" (University Wiscousin Genetic Computer Group-University Research Park 575 Science Drive Suite B, Madisson Wiscousin 53711), un site potentiel de fixation aux lipides pourrait être « Asn Ile Ala Ser Asn Ile Leu Met Thr Gly Cys ».

c- La présence d'une séquence fortement chargée positivement dans l'extrémité C terminale de la séquence peptidique. Il s'agit de la séquence « Arg Arg Arg Val Lys Lys » qui est positionnée aux acides aminés 804-809.

## 5- EXPRESSION DE DIFFERENTES FORMES DU GENE DANS ESCHERICHIA COLI (K12)

Sans expérience complémentaire, il ne peut être déterminé avec certitude, ni la localisation du promoteur responsable de la transcription du gène, ni le début de l'ARN messager codant pour la protéine. En conséquence, il a été choisi d'exprimer les protéines qui commencent par une méthionine, elle même précédée par un site de fixation des ribosomes encore appelé séquence Shine-Dalgarno. Le site de fixation des ribosomes dans Escherichia coli (K12) est décrit dans la littérature, (PNAS (1974), 71 n°4, pp. 1342-1346 et Nucleic Acid Research (1982), 10 n°9, pp. 2971-2996).

Les méthionines qui sont précédées par un site potentiel de fixation aux ribosomes, sont au nombre de quatre, et le tableau III montre leurs positions sur la séquence peptidique ($S_2$) et la séquence nucléotidique ($S_1$).

### TABLEAU III

| | SEQUENCES | |
|---|---|---|
| POSITION METHIONINE | PEPTIDIQUE | NUCLEOTIDIQUE |
| méthionine 1 | 1 | 310-311-312 |
| méthionine 2 | 112 | 642-643-644 |
| méthionine 3 | 160 | 787-788-789 |
| méthionine 4 | 194 | 888-889-890 |

### 5.1- Clonage dans un vecteur d'expression des quatre protéines

A-Description de la technique de la réaction polymérase en chaîne (polymerase chain reaction - PCR)

La technique de la réaction de polymérase en chaîne (PCR) est une méthode bien connue qui permet de copier simultanément les brins d'une séquence d'ADN préalablement dénaturés en utilisant deux oligonucléotides comme amorce [H.A. Erlich, "PCR Technology : Principles and Applications for DNA amplification", (1989), Ed. Macmillan Publishers Ltd, UK, et M.A. Innis et al, "PCR Protocols", (1990), Ed. Academic Press Inc San Diego, Californie 92101, USA]. Le principe de cette technique est résumé ci-après.

De nombreux cycles, dont chacun est constitué de trois étapes, provoquent l'amplification des brins d'ADN d'intérêt ; ces trois étapes sont :

a) dénaturation de la matrice

b) hybridation des amorces sur la matrice

c) extension des amorces.

Après quelques heures de cycles, des centaines de milliers de copies de la matrice originale ont été produites à l'aide d'une ADN polymérase thermostable de Thermus aquaticus, habituellement appelée polymérase Taq. La technique du PCR est basée sur la répétition des trois étapes précédemment citées.

a) *Dénaturation de la matrice* :

L'ADN double brin est dénaturé en ADN simple brin par incubation à haute température (92°C-96°C) pendant approximativement 2 minutes.

b) *Hybridation des amorces* :

Ces amorces sont une paire d'oligonucléotides synthétiques qui s'hybrident avec les extrémités de la région

à amplifier. Les deux amorces s'hybrident avec les brins opposés. Les amorces sont ajoutées en excès, de façon à ce que la formation du complexe amorce - matrice soit favorisée.

c) *Extension des amorces* :

L'étape au cours de laquelle la polymérase Taq assure l'extension du complexe amorce - matrice de 5' vers 3' est effectuée à 72°C.
Dans la technique PCR, le produit d'intérêt apparaît au troisième cycle et il est alors amplifié de manière significative. Au cours du déroulement des cycles, le produit d'amplification devient rapidement la matrice majeure avec laquelle les amorces s'hybrident.

B- Description des amorces utilisées

On a préparé 5 oligonucléotides synthétiques à partir de la séquence de la figure 1.
Le premier oligonucléotide, appelé amorce 1, dont la séquence est la suivante :

**5'-GAT-CCA-TAT-GAC-GGT-CTC-AAC-CGA-AGT-TG-3'**

**Région 1** **Région 2**

SEQ ID N° 3

possède deux régions distinctes. La région 1 est celle qui porte un site de clonage CATATG correspondant au site de reconnaissance de l'endonucléase NdeI et la région 2 celle destinée à s'hybrider avec l'extrémité 5' du gène codant pour la protéine débutant par la méthionine 1.
Le deuxième oligonucléotide est l'amorce 2 dont la séquence est la suivante :

**5'-GAT-CCA-TAT-GCT-GAT-CCA-GCG-ATG-TTT-TGG-GTG-G-3'**

**Région 1** **Région 2**

SEQ ID N° 4

possède deux régions distinctes. La région 1 est celle qui porte un site de clonage CATATG correspondant au site de reconnaissance de l'endonucléase NdeI et la région 2 qui est destinée à s'hybrider avec l'extrémité 5' du gène codant pour la protéine débutant par la méthionine 2.
Le troisième oligonucléotide constituant l'amorce 3, dont la séquence est la suivante :

**5'-GAT-CCA-TAT-GCG-TAA-TTA-TGT-CGA-TGC-TGC-AAT-CG-3'**

**Région 1** **Région 2**

SEQ ID N° 5

possède deux régions distinctes. La région 1 qui porte un site de clonage CATATG correspondant au site de reconnaissance de l'endonucléase NdeI et la région 2 qui est destinée à s'hybrider avec l'extrémité 5' du gène codant pour la protéine débutant par la méthionine 3.
Le quatrième oligonucléotide constitue l'amorce 4 dont la séquence est la suivante :

**5'-GAT-CCA-TAT-GCG-TGA-TGG-TGT-CAG-CAT-TAA-GGA-TTT-TGG-3'**

**Région 1** **Région 2**

SEQ ID N° 6

possède deux régions distinctes. La région 1 est celle qui porte un site de clonage CATATG correspondant au site de reconnaissance de l'endonucléase Ndel et la région 2, destinée à s'hybrider avec l'extrémité 3' du gène codant pour la protéine débutant par la méthionine 4.

Le cinquième oligonucléotide appelé amorce 5 dont la séquence est la suivante :

5'-GAT-CAA-GCT-TAT-CAA-TTC-CCT-GTT-AAT-TGC-AAA-AC-3'

Région 1 Région 2

SEQ ID N° 7

possède deux régions distinctes. La région 1 est celle qui porte un site de clonage AAGCTT correspondant au site de reconnaissance de l'endonucléase HindIII et la région 2, destinée à s'hybrider avec l'extrémité 3' du gène codant pour la protéine.

### 5.2- Obtention de quatre fragments amplifiés correspondants aux quatre protéines

200 ng du plasmide p 838,7 hydrolysés par l'enzyme de restriction EcoRI (NEB) dans les conditions préconisées par le fournisseur sont mélangés à :

- 100 ng de l'amorce 1, 2, 3 ou 4
- 100 ng de l'amorce 5
- 200 μmoles de chacun des quatre désoxynucléotides triphosphates (adénosine, thymidine, cytidine, guanosine)
- 5 μl de mélange réactionnel concentré dix fois (concentration finale 67 mM Tris-HCl, pH 8,8, 16,6 mM $(NH_4)_2SO_4$, 1 mM β-mercaptophénol, 6,7 mM EDTA, 0,15 % Triton®, 200 mg de gélatine).

Le volume est ensuite porté à 50 μl en ajoutant de l'eau.

On ajoute alors 0,5 μl, soit 2,5 unités de Taq polymérase (Beckman® réf. 267301). Après homogénéisation de la solution on recouvre le mélange réactionnel d'huile de paraffine afin d'éviter l'évaporation de la solution aqueuse au cours de la réaction de polymérase en chaîne.

L'amplification se fait au cours de 18 cycles réactionnels dont les étapes d'un cycle sont les suivantes :

1 minute à 92°C → dénaturation
1 minute à 55°C → hybridation
3 minutes à 72°C → polymérisation

Après les 18 cycles prévus, la réaction enzymatique est arrêtée par addition de 20 mM d'EDTA.

Les fragments d'ADN ainsi amplifiés présentent les tailles attendues, qui sont les suivantes :

- amorce 1 : environ 2460 pb
- amorce 2 : environ 2130 pb
- amorce 3 : environ 2000 pb
- amorce 4 : environ 1880 pb

Ces fragments sont ensuite isolés et purifiés sur gel d'agarose 1 %, puis hydrolysés simultanément par les enzymes Ndel et HindIII selon les recommandations du fournisseur (New England Biolabs®) afin de former les extrémités cohésives Ndel et HindIII. Après hydrolyse, le fragment est purifié sur colonne de chromatographie d'exclusion.

### 5.3- Construction des plasmides p868,26 - p869,3 - p886,3 et p88,7

Il s'agit de la construction des vecteurs d'expression des protéines dont les séquences peptidiques correspondantes commencent par les quatre méthionines potentiellement initiales, décrites dans le Tableau III.

Ces quatre plasmides sont préparés à partir du plasmide p466 correspondant à un vecteur de clonage et d'expression de l'ADN complémentaire, de l'urate oxydase d'Aspergillus flavus dans Escherichia coli, décrit dans la demande de brevet EP-0 408 461, qui comprend un fragment du plasmide pBR327 incluant l'origine de réplication et le gène de résistance à l'ampicilline, un promoteur synthétique d'Escherichia coli, une séquence de Shine-Dalgarno, suivie du gène codant pour l'urate-oxydase d'Aspergillus flavus borné par les séquences reconnues par les enzymes Ndel et HindIII, un terminateur de transcription (dérivé du phage fd) et le gène lac i.

Le plasmide p466 est hydrolysé selon les conditions recommandées par le fournisseur par les enzymes Ndel et HindIII, et le fragment portant le gène lac i, le fragment du plasmide pBR327, le promoteur synthétique,

la séquence de Shine-Dalgarno et le terminateur est purifié.

Ce fragment a été ligaturé à chacun des quatre fragments obtenus après la réaction de polymérase en chaîne avec les amorces 1, 2, 3 et 4 en présence de l'amorce 5 préalablement hydrolysée par les endonucléases de restriction NdeI et HindIII afin de libérer les extrémités de ces deux sites, comme il est décrit ci-dessus (5.2). Le produit de ces ligatures a été utilisé pour transformer la souche Escherichia coli RRI (K12) (GIBCO-BRL réf. 5308261 SA). Les transformants obtenus sont analysés.

Le tableau IV montre les transformants retenus, correspondants aux différentes amorces utilisées en accord avec le tableau III, et la Section 5.1, "B-description des amorces utilisées".

## TABLEAU IV

| | CLONE | PLASMIDE |
|---|---|---|
| AMORCE 1 | 868,26 | p868,26 |
| AMORCE 2 | 886,3 | p886,3 |
| AMORCE 3 | 887,7 | p887,7 |
| AMORCE 4 | 869,3 | p869,3 |

### 5.4- Analyse de la protéine produite par chacun des quatre clones

Le schéma général d'étude de l'expression dans ces quatre vecteurs a été le suivant :
Une colonie sert à ensemencer un tube à essai prérempli de milieu LB supplémenté avec 100 µg/ml d'ampicilline. Le tube à essai est alors placé sous agitation une nuit à 37°C.

A partir de cette préculture on ensemence au 1/100 une fiole préremplie du même milieu LB et l'incubation est poursuivie dans les mêmes conditions. Après une heure de culture, l'induction est provoquée par addition de 1 mM d'IPTG (Sigma® I-9003) et l'incubation est poursuivie 3 heures ou 24 heures toujours dans les mêmes conditions de température et d'agitation. Les cultures après expression sont soumises à différentes manipulations :

a) *Préparation des culots bactériens* dont la densité cellulaire correspond à 0,2 ml à DO = 1 (λ = 600 nm) pour analyse sur PAGE-SDS 10 % acrylamide ou 1 ml DO = 3 pour dosage biochimique de l'activité éliminase.

b) *Séparation surnageant/culot bactérien* par centrifugation à 10000 tours/minute pendant 5 minutes à 4°C.

c) *Choc osmotique.* Le choc osmotique est une technique couramment utilisée pour extraire les protéines contenues dans le périplasme des bactéries Gram négatif. Cette technique consiste à faire passer les bactéries d'un milieu à forte pression osmotique dans un milieu à faible pression osmotique. Par exemple Neu et al. dans J. Biol. Chem., (1965) 240, pp. 3685-3692 préconisent l'utilisation d'une solution concentrée de saccharose contenant de l'EDTA comme milieu à forte pression osmotique et de l'eau comme milieu à faible pression osmotique. Dans certains cas particuliers, on a pu extraire également par ce procédé des enzymes cytoplasmiques qui seraient localisées sur la face interne de la membrane cytoplasmique Int. J. Biochem. (1979), 10, pp. 877-883).

Le tableau V résume les résultats obtenus sur les différentes constructions.

## TABLEAU V

| METHIONINE EN POSITION | PLASMIDE | ACTIVITE CULOT | ACTIVITE SURNAGEANT | ACTIVITE CHOC OSMOTIQUE |
|---|---|---|---|---|
| 1 | p868,26 | + | + | + |
| 112 | p886,3 | + | + | + |
| 160 | p887,7 | + | - | non testé |
| 194 | p869,3 | + | - | non testé |

Le plasmide p868,26 produisant en quantité suffisante l'enzyme de fragmentation sous forme soluble dans le surnageant de culture, présente un intérêt spécifique pour l'obtention de fragments majoritairement de 9-10 disaccharides.

**5.5- Transformation des plasmides p868,26 et p886,3 dans les souches MC 1061 et TP 2 339**

La souche bactérienne MC 1061 est décrite dans J. Bacteriol. (1980), 143, n° 2, pp. 971-980, et la souche TP 2339 dans le brevet US-4,945,047.

Les deux souches bactériennes ont été transformées chacune par les deux plasmides p868,26 et p886,3 par la technique décrite dans FMS (Sambrook Fritsch et Maniatis "Molecular cloning : A Laboratory manual" (1989), Ed. Cold Spring Harbor Press (N.Y.) 2ème édition pp. 1:75). Les deux souches transformées ont été cultivées selon la méthode indiquée ci-dessus (5.4- Analyse de la protéine produite par chacun des quatre clones). Les culots bactériens, les surnageants de culture et le choc osmotique ont été dosés sur leur activité éliminasique de la manière suivante :

- les échantillons prélevés ont été dilués selon une gamme, et à chaque 125 µl de chaque dilution effectuée dans le tampon A (décrit dans 2.1- Incubation des pools de bactéries transformées) sont ajoutés 125 µl de N-acétylhéparosane de haute masse moléculaire à la concentration de 20 g/l. L'incubation est de 24 heures et l'analyse des fragments obtenus est effectuée selon le protocole décrit précédemment (2.3- Analyse des produits de fragmentation - Conditions opératoires en HPLC d'exclusion).

Les résultats obtenus sont indiqués dans le tableau VI :

## TABLEAU VI

| | CULOT | | SURNAGEANT | | CHOC OSMOTIQUE | |
|---|---|---|---|---|---|---|
| | 3 heures | 24 heures | 3 heures | 24 heures | 3 heures | 24 heures |
| p868,26/MC1061 | ND* | ND* | ND* | ND* | ND* | + |
| p868,26/TP2339 | ++ | + | ND* | +++ | ++ | ND* |
| p886,3/MC1061 | (+) | ND* | ND* | ND* | + | (+) |
| p886,3/TP2339 | ND* | ND* | ND* | ND* | ND* | ND* |

ND* = Non détectable

Sur ce tableau, on observe que la souche TP 2339 transformée par le plasmide p868,26 (p868,26/TP2339) après 24 heures d'incubation présente une forte activité dans le surnageant de la culture.

**6- OBTENTION DE L'ENZYME RECOMBINEE SOLUBLE**

**6.1- Culture**

Pour obtenir l'enzyme recombinée soluble, une culture de la souche réceptrice TP 2339, qui est une souche d'Escherichia coli RRI (K12) transformée par le plasmide p868,26, est effectuée dans des conditions environnementales favorables à la formation de l'enzyme.

La souche recombinée est conservée en tube glycérol à 20 % à la température de -80°C. Le milieu utilisé est le milieu C est indiqué dans le tableau VII, à la différence que de l'extrait de levure est ajouté à raison de 2 g/l initialement puis 10 g/l après 7 heures de culture. Le fermenteur utilisé est un fermenteur MBR de 18 litres doté de régulateurs de pression d'oxygène, température, pH etc.

*Préculture*

Un tube de lot de semence à -80°C est décongelé et 100 µl sont prélevés et dilués 50 fois puis inoculés dans un flacon à tubulure inférieure prérempli de 500 ml de milieu LB. Le flacon contenant un barreau aimanté

est placé dans un bain thermostaté à 30°C puis relié au fermenteur à inoculer par une aiguille creuse implantée stérilement dans un septum. Le tuyau plastique est inséré dans une pompe péristaltique reliée à un programmateur. Suite à des expériences et des calculs, ce schéma convient pour inoculer un fermenteur de 10 litres de volume utile initial devant démarrer 11 heures après l'inoculation du flacon (par exemple, inoculation du flacon lancé à 17 heures pour inoculation du fermenteur à 4 heures du matin).

*Culture*

Les paramètres de culture sont les suivants :

- pH 7,4
- Pression d'oxygène : 40 mmHg régulée par agitation.
- Température : 37°C
- Surpression 0,1 bar en tête de fermenteur.

D'autres réactifs employés lors de la culture sont les suivants :
$H_2SO_4$ 3N consommation environ 400 ml
KOH 3N consommation environ 1200 ml
Agent antimousse : Struktol® (Schill et Seilacher) type J 673® pur 50 ml. Un apport en glucose en continu est réglé de façon à assurer une concentration en glucose entre 10 et 20 g/l. La quantité en glucose consommée comprenant le glucose initial du milieu est de 1160 g.

La densité optique de la solution mesurée à $\lambda$ = 600 nm a atteint le maximum de sa valeur à 11 heures d'âge de culture, la biomasse est alors d'environ 38 g/l .

*Traitement de la suspension de culture*

La suspension est centrifugée vigoureusement à 14000 g en pots. Le surnageant contient l'enzyme recombinée soluble. Le surnageant est directement utilisable pour, après dilution convenable, fragmenter le N-acétylhéparosane de haute masse moléculaire. Le surnageant peut être également stocké par congélation à -20°C.

**6.2- Dosage de l'activité de l'éliminase**

La mise en évidence de l'activité de l'éliminase soluble recombinante a été effectuée par la fragmentation provoquée d'une quantité de N-acétylhéparosane de haute masse moléculaire.

Comme substrat, ont été utilisés 150 µl de N-acétylhéparosane de haute masse moléculaire purifiés (Préparation) à 20 g/l, solubilisés dans le tampon pH 7,0. (Pour la préparation de ce tampon, dissoudre 100 mmoles de bis-Tris-propane et 200 mmoles de NaCl dans l'eau, et ajuster le pH à 7 avec HCl concentré et compléter à 1 litre).

A ce substrat, ont été ajoutés 150 µl de surnageant contenant l'enzyme recombinée soluble obtenue comme indiqué ci-dessus (6.1- Traitement de la suspension de culture), à différentes dilutions effectuées en tampon pH 7,0. Ce mélange est homogénéisé par agitation puis incubé à 37°C sous agitation dans un agitateur rotatif à 300 tr/min.

La lecture s'effectue après 24 heures d'incubation et la mesure est exprimée en unité éliminase (U.E.). Une unité éliminase est définie comme la quantité d'activité nécessaire pour fragmenter 0,1 mg de N-acétylhéparosane de haute masse moléculaire à la concentration de 10 g/l en N-acétylhéparosane de petite masse moléculaire égale à 5000 Da, en 24 heures, à 37°C, à pH 7,0 en présence de NaCl 200 mM ajoutés.

L'analyse du N-acétylhéparosane fragmenté est effectuée par HPLC d'exclusion selon les conditions décrites dans la Section 2 (2.3- Analyse des produits de fragmentation).

*Calculs*

L'enzyme recombinée soluble dans le volume $V_E$ (µl) dilué d'un facteur $d_E$ à une DO égale à 1 à $\lambda$ = 600 nm (cette densité optique pour l'Escherichia coli (K5) correspond à une biomasse de 400 mg/l) de fin de culture $DO_E$ permet de fragmenter la quantité $Q_{K5}$ (mg) de N-acétylhéparosane de haute masse moléculaire.

L'activité spécifique ($A_{SD}$) de la fin de culture de l'enzyme recombinée soluble s'obtient par la formule :

$$A_{SD} = \frac{10^3 \times d_E \times Q_{K5}(mg) \times 10}{V_E(\mu l) \times DO_E}$$

ce qui donne des UE/ml à DO = 1.

L'activité éliminase de l'enzyme recombinée soluble décrite ci-dessus (6.1) est de 10 U.E.

## TABLEAU VII

**Composition et préparation du milieu C**

**MILIEU C**

Le milieu C est préparé par la réunion des trois solutions stériles ci-dessous :

Solution N°1

dans 700 ml d'eau ultra-purifiée dissoudre dans l'ordre :

Complexant : N'[Tris-(hydroxyméthyl) méthyl] glycine

| | | |
|---|---|---|
| (Tricine commercialisé par Fluka®) | 360 | mg |
| $FeSO_4$, $7H_2O$ | 280 | mg |
| $CaCl_2$, $2H_2O$ | 6,7 | mg |
| $MgCl_2$, $6H_2O$ | 1270 | mg |
| $K_2SO_4$ | 8710 | mg |
| NaCl | 500 | mg |
| KCl | 5000 | mg |
| Hydrolysat de caséine (source principale d'acides aminés) HY CASE SF® (commercialisé par Sheffield) | 25000 | mg |
| Extrait de levure (commercialisé par Difco®) | 18000 | mg |
| Solution d'oligo-éléments (cf. tableau II) | 1 | ml |

Agent antimousse Struktol J673® (commercialisé par Schill et Seilacher) : quelques gouttes à la pipette Pasteur.

Ajuster le pH à 7,4 avec une solution de KOH (d = 1,38) et compléter à 850 ml avec de l'eau ultra-purifiée. Autoclaver le milieu 45 minutes à 120°C.

Solution N°2

Dans environ 40 ml d'eau ultra-purifiée, dissoudre 5 g de $K_2HPO_4$ puis ajuster à 50 ml avec le même solvant. Filtrer la solution obtenue à travers un filtre de porosité 0,2 µm.

Solution N°3

Dissoudre 20,7 g de glucose dans une quantité adéquate d'eau ultra-purifiée et ajuster le volume à 100 ml avec le même solvant. Autoclaver à 110°C pendant 30 minutes.

## 7- SEQUENCE AMINOTERMINALE DE L'ENZYME RECOMBINEE

La souche d'Escherichia coli RRI (K12) a été transformée par le plasmide 868,26 et cultivée comme décrit précédemment (6. Obtention de l'enzyme recombinée soluble ; 6.1 Culture).

Le culot bactérien récupéré par centrifugation a été mis une heure en contact avec une solution de Triton X-100 pour en extraire l'enzyme recombinée sous forme soluble.

Après deux étapes de purification par chromatographie d'échange d'anions sur DEAE Sepharose Fast Flow® et gel filtration sur Superdex 200® (Pharmacia) il a été obtenu un pool présentant les caractéristiques suivantes :

- 0,26 mg/ml de protéines totales
- activité éliminasique sur N-acétylhéparosane de haut PM
- bande majoritaire d'environ 70 000 Da en SDS PAGE

Ce pool a été transmis pour séquençage.

L'échantillon d'enzyme recombinée présumée a été déposé à nouveau sur un gel PAGE SDS 10%. Les protéines ont été transférées sur membrane Problot® (Applied Biosystems) et révélées au bleu de Coomassie. Un doublet de masse moléculaire voisin de 70 000 Da a été détecté.

Les bandes ont été déposées séparément sur un séquenceur Applied Biosystems modèle 476 A. Ces deux bandes présentent la même séquence aminoterminale débutant au niveau du résidu thréonine 2 (Thr2) de la séquence peptidique $S_2$ théorique de l'enzyme recombinée.

Les résultats obtenus en utilisant un programme FSTNML sont donnés dans les tableaux VIII et IX.

### TABLEAU VIII

Bande n° 1 - NUMERO DE SEQUENCE : 112

| Cycles de séquence | Identification HPLC*** | | Conclusion |
|---|---|---|---|
| PTH-AA* N° 1 | Thr | 11 pm** | Thr |
| N° 2 | Val | 20 pm | Val |
| N° 3 | Ser | 4 pm | Ser |
| N° 4 | Thr | 6 pm | Thr |
| N° 5 | Glu | 6 pm | Glu |
| N° 6 | Val | 13 pm | Val |
| N° 7 | Asp | 5 pm | Asp |

## TABLEAU IX

NUMERO DE SEQUENCE : 113

| Cycles de séquence | Identification HPLC*** | | Conclusion |
|---|---|---|---|
| PTH-AA* N° 1 | Thr | 30 pm** | Thr |
| N° 2 | Val | 39 pm | Val |
| N° 3 | Ser | 33 pm | Ser |
| N° 4 | Thr | 14 pm | Thr |
| N° 5 | Glu | 29 pm | Glu |
| N° 6 | Val | 23 pm | Val |
| N° 7 | Asp | 22 pm | Asp |
| N° 8 | His | 10 pm | His |
| N° 9 | Asn | 29 pm | Asn |
| N° 10 | Glu | 15 pm | Glu |

* PTH-AA = phénylthiolhydantoine

** pm = picomolaire

*** Conditions chromatographiques HPLC : chromatographie en phase inverse ; colonne PTH C-18 ® (Biosystems) ; solvant d'élution gradient de (A) et (B) : (A) = tampon acétate 30 mM dans une solution de tétrahydrofurane à 3,5% (pH = 4) (B) = acétonitrile pur, gradient : 0-10mn. B = 10%, 10-18 mn B = 35-38%, 18-23 mn B = 90% ; débit = 0,3 ml/mn ; détection UV λ = 260 nm.

La séquence aminoterminale de l'enzyme recombinée purifiée produite par la souche Escherichia coli RRI (K12) transformée par le plasmide 868,26 correspond au début de la séquence peptidique $S_2$ à l'exception de la formylméthionine aminoterminale. Cet acide aminé a été éliminé par une aminopeptidase (Waller J.P., J.Mol-.Biol., (1963), 7, p483-496).

## EXEMPLES

### EXEMPLE 1

**Fragmentation du N-acétylhéparosane de haute masse moléculaire par l'enzyme recombinée - Obtention d'un N-acétylhéparosane de masse moléculaire d'environ 5000 Da**

On prépare 3 solutions (solutions A,B,C) de N-acétylhéparosane de haute masse moléculaire (Préparation) en dissolvant 20 g de N-acétylhéparosane dans l'eau ultra purifiée, de manière à obtenir après addition de la solution de l'enzyme recombinée, une concentration finale en N-acétylhéparosane de 10 g/l. Comme enzyme, on utilise l'enzyme recombinée soluble. La quantité de l'enzyme recombinée soluble est calculée pour que potentiellement, 15 g de N-acétylhéparosane de haute masse moléculaire puissent être entièrement fragmentés en 24 heures. La réaction enzymatique s'effectue à 37°C à pH 7 et sous agitation douce en présence de 200 mM de NaCl. La durée de la réaction est d'environ 15 heures. Son suivi s'effectue par HPLC d'exclusion

comme il est décrit dans la section 2.3 (2.3- Analyse des produits de fragmentation - Conditions opératoires en HPLC d'exclusion). La réaction est arrêtée par refroidissement. On obtient ainsi 3 lots de N-acétylhéparosane de petite masse moléculaire, les lots A, B et C.

La figure 2 représente les résultats obtenus en HPLC d'exclusion avec les les lots A, B et C.

Les 3 lots de N-acétylhéparosane de petite masse moléculaire obtenus à la fin de la réaction présentent un pic majoritaire qui correspond à 4800 Da évalué par rapport à un étalon standard d'une fraction d'héparine de masse moléculaire de 4800 Da. La proportion de fragments de haute masse moléculaire est négligeable voire nulle à la fin de la réaction enzymatique.

LISTE DE SEQUENCES

(1) INFORMATION GENERALE:

(i) DEPOSANT:
(A) NOM: SANOFI
(B) RUE: 32-34, rue Marbeuf
(C) VILLE: PARIS
(E) PAYS: FRANCE
(F) CODE POSTAL: 75008
(G) TELEPHONE: 61399600
(H) TELECOPIE: 61398637
(I) TELEX: 520405F

(ii) TITRE DE L' INVENTION: Fragment d'ADN portant le gene codant pour l'enzyme de fragmentation du N-acetylheparosane et les sequences adjacentes permettant son expression, enzyme recombinee destinee a la fragmentation du N-acetylheparosane et son utilisation.

(iii) NOMBRE DE SEQUENCES: 7

(iv) FORME LISIBLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release £1.0, Version £1.25 (OEB)

(2) INFORMATION POUR LA SEQ ID NO: 1:

(i) CARACTERISTIQUES DE LA SEQUENCE:
(A) LONGUEUR: 3089 paires de bases
(B) TYPE: acide nucleique
(C) NOMBRE DE BRINS: double
(D) CONFIGURATION: lineaire

(ii) TYPE DE MOLECULE: ADN (genomique)

(iii) HYPOTHETIQUE: NON

(iii) ANTI-SENS: NON

(vi) ORIGINE:
(A) ORGANISME: Escherichia coli
(B) SOUCHE: (K5) SEBR 3282

(xi) DESCRIPTION DE LA SEQUENCE: SEQ ID NO: 1:

GATCCCGGAG TAATTTCATC AAGTGCGATC CCTCCACCAG TGACCTGACG CCTCCCGGCG 60

TGTGAATCCT TTCGGTAAAT CCCTCTTCCA GTGGATAGTG ATACTGCTGC ATCTTAATCT 120

TCTCCATGCA ATAACTGTAT ATTTATACAG TAGCAAATAA TTTGTTTGCT ATCCAGCACG 180

TTTTGCAAAT TACCTGAAAG GTAATATCTA TTCATATTCA CAGTCTTTCT ATCCATATAT 240

GGTTTTTTGG GTAATAGAAT AACCAGATAT GCGGCGCAAC GGGTGCTGCG ACTATCTGGA 300

GATTTAACAT GACGGTCTCA ACCGAAGTTG ACCACAACGA ATACACAGGT AACGGCGTTA 360

CGACATCATT TCCGTATACC TTCCGTATTT TCAAAAAATC CGACCTGGTT GTTCAGGTGT 420

CTGACCTTAA CGGTAACGTT ACAAAACTAG TGCTGGATGC TGGTTATACG GTAACAGGGG 480

CGGGAACTTA TAGTGGCGGT GCAGTGGTTC TTCCGTCGCC GCTTGCTGCT GGCTGGCGAA 540
TCACGATAGA GCGTGTGCTT GATGTGGTGC AGGAGACTGA TCTTCGCAAT CAGGGAAAAT 600
TTTTCCCCGA AGTTCATGAG GATGCATTTG ACTACCTGAC GATGCTGATC CAGCGATGTT 660
TTGGGTGGTT CAGACGTGCA TTGATGAAAC CATCTTTGCT TGCAAAATAT TACGATGCAA 720
AGCAAAACAG AATATCTAAC CTTGCCGATC CATCACTTGA GCAGGACGCT GTAAATAATC 780
GCTCAATGCG TAATTATGTC GATGCTGCAA TCGCCGGAGT TATTGGTGGT TTTGGTTGGT 840
TTATTCAGTA TGGTTCTGGA GCGGTATACA GAACGTTCCA GGATAAGATG CGTGATGGTG 900
TCAGCATTAA GGATTTTGGA GCTCAAAATG GAATCTTAAA TGATAACAAG GATGCTTTTA 960
CAAAATCATT ACATTCGTTT AGCAGTGTTT TTGTTCCGGA AGGGGTATTC AATACATCTT 1020
TAGTTTCTCT TTCACGTTGT GGCTTGTACG GAACAGGTGG GGGAACGATA AAACAGTATG 1080
ACAGAGATGG TAATCATCTG GTTTTTAACA TGCCCGATGG TGGCATGCTT AGTACGCTAA 1140
CAATTATGGG AAATAAATCA GATGATAGTG TGCAGGGACA CCAGGTGTCA TTTTCAGGTG 1200
GCCATGATGT ATCGGTTAAA AATATCAGAT TTACAAATAC GCGAGGACCA GGATTTAGCT 1260
TGATCGCTTA TCCGGATAAT GGTATTCCGT CAGGTTACAT TGTTAGAGAT ATAAGAGGAG 1320
AGTATTTAGG GTTCGCAAAT AATAAAAAAG CAGGTTGTGT GCTTTTTGAT TCATCGCAAA 1380
ATACGCTAAT TGATGGTGTG ATAGCCAGAA ATTATCCTCA GTTTGGTGCA GTGGAACTTA 1440
AAACAGCAGC AAAATATAAC ATTGTCAGCA ATGTTATTGG TGAAGAGTGT CAGCACGTTG 1500
TTTACAATGG AACTGAGACG GAAACTGCCC CAACGAATAA TATCATTAGC AGTGTAATGG 1560
CTAACAACCC AAAATACGCC GCAGTAGTTG TTGGCAAGGG GACTGGTAAC CTGATTTCGG 1620
ATGTGCTGGT TGATTACTCT GAATCGGACG CAAAGCAGGC GCACGGCGTC ACCGTTCAGG 1680
GAAATAATAA TATTGCCAGT AATATTCTAA TGACTGGGTG TGATGGGAAA AATGAATCAG 1740
GAGATCTGCA GACATCTACA ACCATTCGTT TCTTAGATGC TGCACGCAGT AATTATGCGT 1800
CAATATTCCC CATGTATAGT TCTTCCGGCG TGGTTACCTT CGAGGAAGGG TGTATCAGGA 1860
ACTTTGTTGA AATTAAACAT CCGGGTGACA GAAATAATAT TCTGAGTTCT GCATCAGCGG 1920
TGACTGGTAT TTCCAGTATA GACGGCACTA CAAATAGCAA TGTTGTTCAC GTCCCTGCGC 1980
TTGGTCAGTA CGTTGGGACT ATGTCAGGGC GTTTTGAATG GTGGGTTAAA TATTTTAACC 2040
TTGCTAACCA GACGCTTGTT TCTGCAGATA AATTCAGAAT GCTTGCTGAA GGCGATGTAT 2100
CTCTGGCTGT GGGAGGCGGT ATAAGTTCGC AATTGAAATT ATTCAATAGT GATAATACTA 2160
AAGGCACTAT GTCGCTAATA AATGGAAATA TTCGAATATC TACTGGAAAT TCAGAATATA 2220
TACAGTTTTC TGATTCAGCC ATGACACCAT CGACAACGAA TACTTATTCT CTTGGGTTGG 2280
CTGGTCGTGC ATGGTCGGGG GGATTTACCC AGTCAGCGTT TACGGTGCTG TCCGATGCGC 2340
GTTTCAAGAC TGCTCCAGAG GTTATTGATG AGAAAATACT GGACGCATGG GAAAGAGTGG 2400
AATGGGTTTC ATACCAGTAC CTTGACAGGA TCGAAGTGAA AGGTAAAGAC GGAGCAAGAT 2460
GGCACTTTGG TGCAGTTGCG CAGCATGTTA TCAGTGTATT TCAGAATGAA GGCATAGATG 2520

TGTCACGACT GGCATTTATC TGTTATGACA AGTGGAATGA GACCCCGGCA GAATACAGGG 2580

ATGTGACGGA AGAAGAGCAT TCTGCAGGAG TTTACCCACT TATACAGACA AAGGTTCTGG 2640

TACGCGAAGC CGTCGAGGCT GGTGAATGTT ACGGTATCCG TTATGAAGAG GCTCTGATTC 2700

TGGAATCTGC GATGATGAGA CGCAGGGTTA AAAAGCTGGA AGAGCAAGTT TTGCAATTAA 2760

CAGGGAATTG AACCGTAAAT GGTGTGTTGT TGCGCGGTAT ACTTTTCCTG AAGCAGGGTG 2820

TTTGCAAATA AACGGGTTTC GTTATGTCAT TCCAACTAAC CAATGAAACT TCAAATCAGT 2880

GGCTTAGTGT TAGTTCTCTT GCTGCGGTTA TTGCAGGTGT CCCTCCGGAG GTTGCTTTGG 2940

GGGCTTTGGC TGGGGCGGTA ATTTTTGTTA CCTCTGCGGT AGAGTATCCT ATTCGTCGTC 3000

GTGTACTCTT GTCGATGCTT AGCTTTCTCT GCGGCCTTCT TTTTTATAAA CCAGCAGCAT 3060

CAATTCTTAT CGGCATAGCC AGCCTGATC 3089

(2) INFORMATION POUR LA SEQ ID NO: 2:

(i) CARACTERISTIQUES DE LA SEQUENCE:
(A) LONGUEUR: 820 acides amines
(B) TYPE: acide amine
(C) NOMBRE DE BRINS: simple
(D) CONFIGURATION: lineaire

(ii) TYPE DE MOLECULE: proteine

(xi) DESCRIPTION DE LA SEQUENCE: SEQ ID NO: 2:

Met Thr Val Ser Thr Glu Val Asp His Asn Glu Tyr Thr Gly Asn Gly
1 5 10 15

Val Thr Thr Ser Phe Pro Tyr Thr Phe Arg Ile Phe Lys Lys Ser Asp
20 25 30

Leu Val Val Gln Val Ser Asp Leu Asn Gly Asn Val Thr Lys Leu Val
35 40 45

Leu Asp Ala Gly Tyr Thr Val Thr Gly Ala Gly Thr Tyr Ser Gly Gly
50 55 60

Ala Val Val Leu Pro Ser Pro Leu Ala Ala Gly Trp Arg Ile Thr Ile
65 70 75 80

Glu Arg Val Leu Asp Val Val Gln Glu Thr Asp Leu Arg Asn Gln Gly
85 90 95

Lys Phe Phe Pro Glu Val His Glu Asp Ala Phe Asp Tyr Leu Thr Met
100 105 110

Leu Ile Gln Arg Cys Phe Gly Trp Phe Arg Arg Ala Leu Met Lys Pro
115 120 125

Ser Leu Leu Ala Lys Tyr Tyr Asp Ala Lys Gln Asn Arg Ile Ser Asn
130 135 140

Leu Ala Asp Pro Ser Leu Glu Gln Asp Ala Val Asn Asn Arg Ser Met
145 150 155 160

Arg Asn Tyr Val Asp Ala Ala Ile Ala Gly Val Ile Gly Gly Phe Gly
165 170 175

```
Trp Phe Ile Gln Tyr Gly Ser Gly Ala Val Tyr Arg Thr Phe Gln Asp
            180                 185                 190

Lys Met Arg Asp Gly Val Ser Ile Lys Asp Phe Gly Ala Gln Asn Gly
        195                 200                 205

Ile Leu Asn Asp Asn Lys Asp Ala Phe Thr Lys Ser Leu His Ser Phe
    210                 215                 220

Ser Ser Val Phe Val Pro Glu Gly Val Phe Asn Thr Ser Leu Val Ser
225                 230                 235                 240

Leu Ser Arg Cys Gly Leu Tyr Gly Thr Gly Gly Gly Thr Ile Lys Gln
                245                 250                 255

Tyr Asp Arg Asp Gly Asn His Leu Val Phe Asn Met Pro Asp Gly Gly
            260                 265                 270

Met Leu Ser Thr Leu Thr Ile Met Gly Asn Lys Ser Asp Asp Ser Val
        275                 280                 285

Gln Gly His Gln Val Ser Phe Ser Gly Gly His Asp Val Ser Val Lys
    290                 295                 300

Asn Ile Arg Phe Thr Asn Thr Arg Gly Pro Gly Phe Ser Leu Ile Ala
305                 310                 315                 320

Tyr Pro Asp Asn Gly Ile Pro Ser Gly Tyr Ile Val Arg Asp Ile Arg
                325                 330                 335

Gly Glu Tyr Leu Gly Phe Ala Asn Asn Lys Lys Ala Gly Cys Val Leu
            340                 345                 350

Phe Asp Ser Ser Gln Asn Thr Leu Ile Asp Gly Val Ile Ala Arg Asn
        355                 360                 365

Tyr Pro Gln Phe Gly Ala Val Glu Leu Lys Thr Ala Ala Lys Tyr Asn
    370                 375                 380

Ile Val Ser Asn Val Ile Gly Glu Glu Cys Gln His Val Val Tyr Asn
385                 390                 395                 400

Gly Thr Glu Thr Glu Thr Ala Pro Thr Asn Asn Ile Ile Ser Ser Val
                405                 410                 415

Met Ala Asn Asn Pro Lys Tyr Ala Ala Val Val Val Gly Lys Gly Thr
            420                 425                 430

Gly Asn Leu Ile Ser Asp Val Leu Val Asp Tyr Ser Glu Ser Asp Ala
        435                 440                 445

Lys Gln Ala His Gly Val Thr Val Gln Gly Asn Asn Asn Ile Ala Ser
    450                 455                 460

Asn Ile Leu Met Thr Gly Cys Asp Gly Lys Asn Glu Ser Gly Asp Leu
465                 470                 475                 480

Gln Thr Ser Thr Thr Ile Arg Phe Leu Asp Ala Ala Arg Ser Asn Tyr
                485                 490                 495

Ala Ser Ile Phe Pro Met Tyr Ser Ser Ser Gly Val Val Thr Phe Glu
            500                 505                 510

Glu Gly Cys Ile Arg Asn Phe Val Glu Ile Lys His Pro Gly Asp Arg
        515                 520                 525
```

```
Asn Asn Ile Leu Ser Ser Ala Ser Ala Val Thr Gly Ile Ser Ser Ile
    530                 535                 540

Asp Gly Thr Thr Asn Ser Asn Val Val His Val Pro Ala Leu Gly Gln
545                 550                 555                 560

Tyr Val Gly Thr Met Ser Gly Arg Phe Glu Trp Trp Val Lys Tyr Phe
                565                 570                 575

Asn Leu Ala Asn Gln Thr Leu Val Ser Ala Asp Lys Phe Arg Met Leu
            580                 585                 590

Ala Glu Gly Asp Val Ser Leu Ala Val Gly Gly Gly Ile Ser Ser Gln
        595                 600                 605

Leu Lys Leu Phe Asn Ser Asp Asn Thr Lys Gly Thr Met Ser Leu Ile
    610                 615                 620

Asn Gly Asn Ile Arg Ile Ser Thr Gly Asn Ser Glu Tyr Ile Gln Phe
625                 630                 635                 640

Ser Asp Ser Ala Met Thr Pro Ser Thr Thr Asn Thr Tyr Ser Leu Gly
                645                 650                 655

Leu Ala Gly Arg Ala Trp Ser Gly Gly Phe Thr Gln Ser Ala Phe Thr
            660                 665                 670

Val Leu Ser Asp Ala Arg Phe Lys Thr Ala Pro Glu Val Ile Asp Glu
        675                 680                 685

Lys Ile Leu Asp Ala Trp Glu Arg Val Glu Trp Val Ser Tyr Gln Tyr
    690                 695                 700

Leu Asp Arg Ile Glu Val Lys Gly Lys Asp Gly Ala Arg Trp His Phe
705                 710                 715                 720

Gly Ala Val Ala Gln His Val Ile Ser Val Phe Gln Asn Glu Gly Ile
                725                 730                 735

Asp Val Ser Arg Leu Ala Phe Ile Cys Tyr Asp Lys Trp Asn Glu Thr
            740                 745                 750

Pro Ala Glu Tyr Arg Asp Val Thr Glu Glu Glu His Ser Ala Gly Val
        755                 760                 765

Tyr Pro Leu Ile Gln Thr Lys Val Leu Val Arg Glu Ala Val Glu Ala
    770                 775                 780

Gly Glu Cys Tyr Gly Ile Arg Tyr Glu Glu Ala Leu Ile Leu Glu Ser
785                 790                 795                 800

Ala Met Met Arg Arg Arg Val Lys Lys Leu Glu Glu Gln Val Leu Gln
                805                 810                 815

Leu Thr Gly Asn
            820
```

(2) INFORMATION POUR LA SEQ ID NO: 3:

(i) CARACTERISTIQUES DE LA SEQUENCE:
(A) LONGUEUR: 29 paires de bases
(B) TYPE: acide nucleique
(C) NOMBRE DE BRINS: simple
(D) CONFIGURATION: lineaire

(ii) TYPE DE MOLECULE: Oligonucleotide

(xi) DESCRIPTION DE LA SEQUENCE: SEQ ID NO: 3:

GATCCATATG ACGGTCTCAA CCGAAGTTG 29

(2) INFORMATION POUR LA SEQ ID NO: 4:

(i) CARACTERISTIQUES DE LA SEQUENCE:
(A) LONGUEUR: 34 paires de bases
(B) TYPE: acide nucleique
(C) NOMBRE DE BRINS: simple
(D) CONFIGURATION: lineaire

(ii) TYPE DE MOLECULE: Oligonucleotide

(xi) DESCRIPTION DE LA SEQUENCE: SEQ ID NO: 4:

GATCCATATG CTGATCCAGC GATGTTTTGG GTGG 34

(2) INFORMATION POUR LA SEQ ID NO: 5:

(i) CARACTERISTIQUES DE LA SEQUENCE:
(A) LONGUEUR: 35 paires de bases
(B) TYPE: acide nucleique
(C) NOMBRE DE BRINS: simple
(D) CONFIGURATION: lineaire

(ii) TYPE DE MOLECULE: Oligonucleotide

(xi) DESCRIPTION DE LA SEQUENCE: SEQ ID NO: 5:

GATCCATATG CGTAATTATG TCGATGCTGC AATCG 35

(2) INFORMATION POUR LA SEQ ID NO: 6:

(i) CARACTERISTIQUES DE LA SEQUENCE:
(A) LONGUEUR: 39 paires de bases
(B) TYPE: acide nucleique
(C) NOMBRE DE BRINS: simple
(D) CONFIGURATION: lineaire

(ii) TYPE DE MOLECULE: Oligonucleotide

(xi) DESCRIPTION DE LA SEQUENCE: SEQ ID NO: 6:

GATCCATATG CGTGATGGTG TCAGCATTAA GGATTTTGG 39

(2) INFORMATION POUR LA SEQ ID NO: 7:

(i) CARACTERISTIQUES DE LA SEQUENCE:
(A) LONGUEUR: 35 paires de bases
(B) TYPE: acide nucleique
(C) NOMBRE DE BRINS: simple
(D) CONFIGURATION: lineaire

(ii) TYPE DE MOLECULE: Oligonucleotide

(xi) DESCRIPTION DE LA SEQUENCE: SEQ ID NO: 7:

GATCAAGCTT ATCAATTCCC TGTTAATTGC AAAAC 35

# FIGURE 1

## SEQUENCE NUCLEOTIDIQUE DOUBLE BRIN, ADDITIONNEE DE LA PHASE DE LECTURE DE 820 ACIDES AMINES.

```
GATCCCGGAG TAATTTCATC AAGTGCGATC CCTCCACCAG TGACCTGACG
- - - - - - - - - - - - - - - - - - - - - - - - - - - -     50
CTAGGGCCTC ATTAAAGTAG TTCACGCTAG GGAGGTGGTC ACTGGACTGC

CCTCCCGGCG TGTGAATCCT TTCGGTAAAT CCCTCTTCCA GTGGATAGTG
- - - - - - - - - - - - - - - - - - - - - - - - - - - -     100
GGAGGGCCGC ACACTTAGGA AAGCCATTTA GGGAGAAGGT CACCTATCAC

ATACTGCTGC ATCTTAATCT TCTCCATGCA ATAACTGTAT ATTTATACAG
- - - - - - - - - - - - - - - - - - - - - - - - - - - -     150
TATGACGACG TAGAATTAGA AGAGGTACGT TATTGACATA TAAATATGTC

TAGCAAATAA TTTGTTTGCT ATCCAGCACG TTTTGCAAAT TACCTGAAAG
- - - - - - - - - - - - - - - - - - - - - - - - - - - -     200
ATCGTTTATT AAACAAACGA TAGGTCGTGC AAAACGTTTA ATGGACTTTC

GTAATATCTA TTCATATTCA CAGTCTTTCT ATCCATATAT GGTTTTTTGG
- - - - - - - - - - - - - - - - - - - - - - - - - - - -     250
CATTATAGAT AAGTATAAGT GTCAGAAAGA TAGGTATATA CCAAAAAACC

GTAATAGAAT AACCAGATAT GCGGCGCAAC GGGTGCTGCG ACTATCTGGA
- - - - - - - - - - - - - - - - - - - - - - - - - - - -     300
CATTATCTTA TTGGTCTATA CGCCGCGTTG CCCACGACGC TGATAGACCT

GA TTT AAC ATG ACG GTC TCA ACC GAA GTT GAC CAC AAC GAA TAC
- - - - - - - - - - - - - - - - - - - - - - - - - - - - - - - 344
CT AAA TTG TAC TGC CAG AGT TGG CTT CAA CTG GTG TTG CTT ATG
           Met Thr Val Ser Thr Glu Val Asp His Asn Glu Tyr
            1               5                  10

ACA GGT AAC GGC GTT ACG ACA TCA TTT CCG TAT ACC TTC CGT ATT
- - - - - - - - - - - - - - - - - - - - - - - - - - - - - - 389
TGT CCA TTG CCG CAA TGC TGT AGT AAA GGC ATA TGG AAG GCA TAA
Thr Gly Asn Gly Val Thr Thr Ser Phe Pro Tyr Thr Phe Arg Ile
        15                  20                  25

TTC AAA AAA TCC GAC CTG GTT GTT CAG GTG TCT GAC CTT AAC GGT
- - - - - - - - - - - - - - - - - - - - - - - - - - - - - - 434
AAG TTT TTT AGG CTG GAC CAA CAA GTC CAC AGA CTG GAA TTG CCA
Phe Lys Lys Ser Asp Leu Val Val Gln Val Ser Asp Leu Asn Gly
        30                  35                  40

AAC GTT ACA AAA CTA GTG CTG GAT GCT GGT TAT ACG GTA ACA GGG
- - - - - - - - - - - - - - - - - - - - - - - - - - - - - - 479
TTG CAA TGT TTT GAT CAC GAC CTA CGA CCA ATA TGC CAT TGT CCC
Asn Val Thr Lys Leu Val Leu Asp Ala Gly Tyr Thr Val Thr Gly
        45                  50                  55

GCG GGA ACT TAT AGT GGC GGT GCA GTG GTT CTT CCG TCG CCG CTT
- - - - - - - - - - - - - - - - - - - - - - - - - - - - - - 524
CGC CCT TGA ATA TCA CCG CCA CGT CAC CAA GAA GGC AGC GGC GAA
Ala Gly Thr Tyr Ser Gly Gly Ala Val Val Leu Pro Ser Pro Leu
        60                  65                  70
```

```
GCT GCT GGC TGG CGA ATC ACG ATA GAG CGT GTG CTT GAT GTG GTG
- - - - - - - - - - - - - - - - - - - - - - - - - - - - - - 569
CGA CGA CCG ACC GCT TAG TGC TAT CTC GCA CAC GAA CTA CAC CAC
Ala Ala Gly Trp Arg Ile Thr Ile Glu Arg Val Leu Asp Val Val
         75                  80                  85

CAG GAG ACT GAT CTT CGC AAT CAG GGA AAA TTT TTC CCC GAA GTT
- - - - - - - - - - - - - - - - - - - - - - - - - - - - - - 614
GTC CTC TGA CTA GAA GCG TTA GTC CCT TTT AAA AAG GGG CTT CAA
Gln Glu Thr Asp Leu Arg Asn Gln Gly Lys Phe Phe Pro Glu Val
         90                  95                 100

CAT GAG GAT GCA TTT GAC TAC CTG ACG ATG CTG ATC CAG CGA TGT
- - - - - - - - - - - - - - - - - - - - - - - - - - - - - - 659
GTA CTC CTA CGT AAA CTG ATG GAC TGC TAC GAC TAG GTC GCT ACA
His Glu Asp Ala Phe Asp Tyr Leu Thr Met Leu Ile Gln Arg Cys
        105                 110                 115

TTT GGG TGG TTC AGA CGT GCA TTG ATG AAA CCA TCT TTG CTT GCA
- - - - - - - - - - - - - - - - - - - - - - - - - - - - - - 704
AAA CCC ACC AAG TCT GCA CGT AAC TAC TTT GGT AGA AAC GAA CGT
Phe Gly Trp Phe Arg Arg Ala Leu Met Lys Pro Ser Leu Leu Ala
        120                 125                 130

AAA TAT TAC GAT GCA AAG CAA AAC AGA ATA TCT AAC CTT GCC GAT
- - - - - - - - - - - - - - - - - - - - - - - - - - - - - - 749
TTT ATA ATG CTA CGT TTC GTT TTG TCT TAT AGA TTG GAA CGG CTA
Lys Tyr Tyr Asp Ala Lys Gln Asn Arg Ile Ser Asn Leu Ala Asp
        135                 140                 145

CCA TCA CTT GAG CAG GAC GCT GTA AAT AAT CGC TCA ATG CGT AAT
- - - - - - - - - - - - - - - - - - - - - - - - - - - - - - 794
GGT AGT GAA CTC GTC CTG CGA CAT TTA TTA GCG AGT TAC GCA TTA
Pro Ser Leu Glu Gln Asp Ala Val Asn Asn Arg Ser Met Arg Asn
        150                 155                 160

TAT GTC GAT GCT GCA ATC GCC GGA GTT ATT GGT GGT TTT GGT TGG
- - - - - - - - - - - - - - - - - - - - - - - - - - - - - - 839
ATA CAG CTA CGA CGT TAG CGG CCT CAA TAA CCA CCA AAA CCA ACC
Tyr Val Asp Ala Ala Ile Ala Gly Val Ile Gly Gly Phe Gly Trp
        165                 170                 175

TTT ATT CAG TAT GGT TCT GGA GCG GTA TAC AGA ACG TTC CAG GAT
- - - - - - - - - - - - - - - - - - - - - - - - - - - - - - 884
AAA TAA GTC ATA CCA AGA CCT CGC CAT ATG TCT TGC AAG GTC CTA
Phe Ile Gln Tyr Gly Ser Gly Ala Val Tyr Arg Thr Phe Gln Asp
        180                 185                 190

AAG ATG CGT GAT GGT GTC AGC ATT AAG GAT TTT GGA GCT CAA AAT
- - - - - - - - - - - - - - - - - - - - - - - - - - - - - - 929
TTC TAC GCA CTA CCA CAG TCG TAA TTC CTA AAA CCT CGA GTT TTA
Lys Met Arg Asp Gly Val Ser Ile Lys Asp Phe Gly Ala Gln Asn
        195                 200                 205

GGA ATC TTA AAT GAT AAC AAG GAT GCT TTT ACA AAA TCA TTA CAT
- - - - - - - - - - - - - - - - - - - - - - - - - - - - - - 974
CCT TAG AAT TTA CTA TTG TTC CTA CGA AAA TGT TTT AGT AAT GTA
Gly Ile Leu Asn Asp Asn Lys Asp Ala Phe Thr Lys Ser Leu His
        210                 215                 220
```

```
TCG TTT AGC AGT GTT TTT GTT CCG GAA GGG GTA TTC AAT ACA TCT
--------------------------------------------------------- 1019
AGC AAA TCG TCA CAA AAA CAA GGC CTT CCC CAT AAG TTA TGT AGA
Ser Phe Ser Ser Val Phe Val Pro Glu Gly Val Phe Asn Thr Ser
        225                 230                 235

TTA GTT TCT CTT TCA CGT TGT GGC TTG TAC GGA ACA GGT GGG GGA
--------------------------------------------------------- 1064
AAT CAA AGA GAA AGT GCA ACA CCG AAC ATG CCT TGT CCA CCC CCT
Leu Val Ser Leu Ser Arg Cys Gly Leu Tyr Gly Thr Gly Gly Gly
        240                 245                 250

ACG ATA AAA CAG TAT GAC AGA GAT GGT AAT CAT CTG GTT TTT AAC
--------------------------------------------------------- 1109
TGC TAT TTT GTC ATA CTG TCT CTA CCA TTA GTA GAC CAA AAA TTG
Thr Ile Lys Gln Tyr Asp Arg Asp Gly Asn His Leu Val Phe Asn
        255                 260                 265

ATG CCC GAT GGT GGC ATG CTT AGT ACG CTA ACA ATT ATG GGA AAT
--------------------------------------------------------- 1154
TAC GGG CTA CCA CCG TAC GAA TCA TGC GAT TGT TAA TAC CCT TTA
Met Pro Asp Gly Gly Met Leu Ser Thr Leu Thr Ile Met Gly Asn
        270                 275                 280

AAA TCA GAT GAT AGT GTG CAG GgA CAC CAG GTG TCA TTT TCA GGT
--------------------------------------------------------- 1199
TTT AGT CTA CTA TCA CAC GTC CcT GTG GTC CAC AGT AAA AGT CCA
Lys Ser Asp Asp Ser Val Gln Gly His Gln Val Ser Phe Ser Gly
        285                 290                 295

GGC CAT GAT GTA TCG GTT AAA AAT ATC AGA TTT ACA AAT ACG CGA
--------------------------------------------------------- 1244
CCG GTA CTA CAT AGC CAA TTT TTA TAG TCT AAA TGT TTA TGC GCT
Gly His Asp Val Ser Val Lys Asn Ile Arg Phe Thr Asn Thr Arg
        300                 305                 310

GGA CCA GGA TTT AGC TTG ATC GCT TAT CCG GAT AAT GGT ATT CCG
--------------------------------------------------------- 1289
CCT GGT CCT AAA TCG AAC TAG CGA ATA GGC CTA TTA CCA TAA GGC
Gly Pro Gly Phe Ser Leu Ile Ala Tyr Pro Asp Asn Gly Ile Pro
        315                 320                 325

TCA GGT TAC ATT GTT AGA GAT ATA AGA GGA GAG TAT TTA GGG TTC
--------------------------------------------------------- 1334
AGT CCA ATG TAA CAA TCT CTA TAT TCT CCT CTC ATA AAT CCC AAG
Ser Gly Tyr Ile Val Arg Asp Ile Arg Gly Glu Tyr Leu Gly Phe
        330                 335                 340

GCA AAT AAT AAA AAA GCA GGT TGT GTG CTT TTT GAT TCA TCG CAA
--------------------------------------------------------- 1379
CGT TTA TTA TTT TTT CGT CCA ACA CAC GAA AAA CTA AGT AGC GTT
Ala Asn Asn Lys Lys Ala Gly Cys Val Leu Phe Asp Ser Ser Gln
        345                 350                 355

AAT ACG CTA ATT GAT GGT GTG ATA GCC AGA AAT TAT CCT CAG TTT
--------------------------------------------------------- 1424
TTA TGC GAT TAA CTA CCA CAC TAT CGG TCT TTA ATA GGA GTC AAA
Asn Thr Leu Ile Asp Gly Val Ile Ala Arg Asn Tyr Pro Gln Phe
        360                 365                 370
```

```
GGT GCA GTG GAA CTT AAA ACA GCA GCA AAA TAT AAC ATT GTC AGC
- - - - - - - - - - - - - - - - - - - - - - - - - - - - - - 1469
CCA CGT CAC CTT GAA TTT TGT CGT CGT TTT ATA TTG TAA CAG TCG
Gly Ala Val Glu Leu Lys Thr Ala Ala Lys Tyr Asn Ile Val Ser
        375                 380                 385

AAT GTT ATT GGT GAA GAG TGT CAG CAC GTT GTT TAC AAT GGA ACT
- - - - - - - - - - - - - - - - - - - - - - - - - - - - - - 1514
TTA CAA TAA CCA CTT CTC ACA GTC GTG CAA CAA ATG TTA CCT TGA
Asn Val Ile Gly Glu Glu Cys Gln His Val Val Tyr Asn Gly Thr
        390                 395                 400

GAG ACG GAA ACT GCC CCA ACG AAT AAT ATC ATT AGC AGT GTA ATG
- - - - - - - - - - - - - - - - - - - - - - - - - - - - - - 1559
CTC TGC CTT TGA CGG GGT TGC TTA TTA TAG TAA TCG TCA CAT TAC
Glu Thr Glu Thr Ala Pro Thr Asn Asn Ile Ile Ser Ser Val Met
        405                 410                 415

GCT AAC AAC CCA AAA TAC GCC GCA GTA GTT GTT GGC AAG GGG ACT
- - - - - - - - - - - - - - - - - - - - - - - - - - - - - - 1604
CGA TTG TTG GGT TTT ATG CGG CGT CAT CAA CAA CCG TTC CCC TGA
Ala Asn Asn Pro Lys Tyr Ala Ala Val Val Val Gly Lys Gly Thr
        420                 425                 430

GGT AAC CTG ATT TCG GAT GTG CTG GTT GAT TAC TCT GAA TCG GAC
- - - - - - - - - - - - - - - - - - - - - - - - - - - - - - 1649
CCA TTG GAC TAA AGC CTA CAC GAC CAA CTA ATG AGA CTT AGC CTG
Gly Asn Leu Ile Ser Asp Val Leu Val Asp Tyr Ser Glu Ser Asp
        435                 440                 445

GCA AAG CAG GCG CAC GGC GTC ACC GTT CAG GGA AAT AAT AAT ATT
- - - - - - - - - - - - - - - - - - - - - - - - - - - - - - 1694
CGT TTC GTC CGC GTG CCG CAG TGG CAA GTC CCT TTA TTA TTA TAA
Ala Lys Gln Ala His Gly Val Thr Val Gln Gly Asn Asn Asn Ile
        450                 455                 460

GCC AGT AAT ATT CTA ATG ACT GGG TGT GAT GGG AAA AAT GAA TCA
- - - - - - - - - - - - - - - - - - - - - - - - - - - - - - 1739
CGG TCA TTA TAA GAT TAC TGA CCC ACA CTA CCC TTT TTA CTT AGT
Ala Ser Asn Ile Leu Met Thr Gly Cys Asp Gly Lys Asn Glu Ser
        465                 470                 475

GGA GAT CTG CAG ACA TCT ACA ACC ATT CGT TTC TTA GAT GCT GCA
- - - - - - - - - - - - - - - - - - - - - - - - - - - - - - 1784
CCT CTA GAC GTC TGT AGA TGT TGG TAA GCA AAG AAT CTA CGA CGT
Gly Asp Leu Gln Thr Ser Thr Thr Ile Arg Phe Leu Asp Ala Ala
        480                 485                 490

CGC AGT AAT TAT GCG TCA ATA TTC CCC ATG TAT AGT TCT TCC GGC
- - - - - - - - - - - - - - - - - - - - - - - - - - - - - - 1829
GCG TCA TTA ATA CGC AGT TAT AAG GGG TAC ATA TCA AGA AGG CCG
Arg Ser Asn Tyr Ala Ser Ile Phe Pro Met Tyr Ser Ser Ser Gly
        495                 500                 505

GTG GTT ACC TTC GAG GAA GGG TGT ATC AGG AAC TTT GTT GAA ATT
- - - - - - - - - - - - - - - - - - - - - - - - - - - - - - 1874
CAC CAA TGG AAG CTC CTT CCC ACA TAG TCC TTG AAA CAA CTT TAA
Val Val Thr Phe Glu Glu Gly Cys Ile Arg Asn Phe Val Glu Ile
        510                 515                 520
```

```
AAA CAT CCG GGT GAC AGA AAT AAT ATT CTG AGT TCT GCA TCA GCG
- - - - - - - - - - - - - - - - - - - - - - - - - - - - - - 1919
TTT GTA GGC CCA CTG TCT TTA TTA TAA GAC TCA AGA CGT AGT CGC
Lys His Pro Gly Asp Arg Asn Asn Ile Leu Ser Ser Ala Ser Ala
        525                 530                 535

GTG ACT GGT ATT TCC AGT ATA GAC GGC ACT ACA AAT AGC AAT GTT
- - - - - - - - - - - - - - - - - - - - - - - - - - - - - - 1964
CAC TGA CCA TAA AGG TCA TAT CTG CCG TGA TGT TTA TCG TTA CAA
Val Thr Gly Ile Ser Ser Ile Asp Gly Thr Thr Asn Ser Asn Val
        540                 545                 550

GTT CAC GTC CCT GCG CTT GGT CAG TAC GTT GGG ACT ATG TCA GGG
- - - - - - - - - - - - - - - - - - - - - - - - - - - - - - 2009
CAA GTG CAG GGA CGC GAA CCA GTC ATG CAA CCC TGA TAC AGT CCC
Val His Val Pro Ala Leu Gly Gln Tyr Val Gly Thr Met Ser Gly
        555                 560                 565

CGT TTT GAA TGG TGG GTT AAA TAT TTT AAC CTT GCT AAC CAG ACG
- - - - - - - - - - - - - - - - - - - - - - - - - - - - - - 2054
GCA AAA CTT ACC ACC CAA TTT ATA AAA TTG GAA CGA TTG GTC TGC
Arg Phe Glu Trp Trp Val Lys Tyr Phe Asn Leu Ala Asn Gln Thr
        570                 575                 580

CTT GTT TCT GCA GAT AAA TTC AGA ATG CTT GCT GAA GGC GAT GTA
- - - - - - - - - - - - - - - - - - - - - - - - - - - - - - 2099
GAA CAA AGA CGT CTA TTT AAG TCT TAC GAA CGA CTT CCG CTA CAT
Leu Val Ser Ala Asp Lys Phe Arg Met Leu Ala Glu Gly Asp Val
        585                 590                 595

TCT CTG GCT GTG GGA GGC GGT ATA AGT TCG CAA TTG AAA TTA TTC
- - - - - - - - - - - - - - - - - - - - - - - - - - - - - - 2144
AGA GAC CGA CAC CCT CCG CCA TAT TCA AGC GTT AAC TTT AAT AAG
Ser Leu Ala Val Gly Gly Gly Ile Ser Ser Gln Leu Lys Leu Phe
        600                 605                 610

AAT AGT GAT AAT ACT AAA GGC ACT ATG TCG CTA ATA AAT GGA AAT
- - - - - - - - - - - - - - - - - - - - - - - - - - - - - - 2189
TTA TCA CTA TTA TGA TTT CCG TGA TAC AGC GAT TAT TTA CCT TTA
Asn Ser Asp Asn Thr Lys Gly Thr Met Ser Leu Ile Asn Gly Asn
        615                 620                 625

ATT CGA ATA TCT ACT GGA AAT TCA GAA TAT ATA CAG TTT TCT GAT
- - - - - - - - - - - - - - - - - - - - - - - - - - - - - - 2234
TAA GCT TAT AGA TGA CCT TTA AGT CTT ATA TAT GTC AAA AGA CTA
Ile Arg Ile Ser Thr Gly Asn Ser Glu Tyr Ile Gln Phe Ser Asp
        630                 635                 640

TCA GCC ATG ACA CCA TCG ACA ACG AAT ACT TAT TCT CTT GGG TTG
- - - - - - - - - - - - - - - - - - - - - - - - - - - - - - 2279
AGT CGG TAC TGT GGT AGC TGT TGC TTA TGA ATA AGA GAA CCC AAC
Ser Ala Met Thr Pro Ser Thr Thr Asn Thr Tyr Ser Leu Gly Leu
        645                 650                 655

GCT GGT CGT GCA TGG TCG GGG GGA TTT ACC CAG TCA GCG TTT ACG
- - - - - - - - - - - - - - - - - - - - - - - - - - - - - - 2324
CGA CCA GCA CGT ACC AGC CCC CCT AAA TGG GTC AGT CGC AAA TGC
Ala Gly Arg Ala Trp Ser Gly Gly Phe Thr Gln Ser Ala Phe Thr
        660                 665                 670
```

```
GTG CTG TCC GAT GCG CGT TTC AAG ACT GCT CCA GAG GTT ATT GAT
--------------------------------------------------------- 2369
CAC GAC AGG CTA CGC GCA AAG TTC TGA CGA GGT CTC CAA TAA CTA
Val Leu Ser Asp Ala Arg Phe Lys Thr Ala Pro Glu Val Ile Asp
        675                 680                 685

GAG AAA ATA CTG GAC GCA TGG GAA AGA GTG GAA TGG GTT TCA TAC
--------------------------------------------------------- 2414
CTC TTT TAT GAC CTG CGT ACC CTT TCT CAC CTT ACC CAA AGT ATG
Glu Lys Ile Leu Asp Ala Trp Glu Arg Val Glu Trp Val Ser Tyr
        690                 695                 700

CAG TAC CTT GAC AGG ATC GAA GTG AAA GGT AAA GAC GGA GCA AGA
--------------------------------------------------------- 2459
GTC ATG GAA CTG TCC TAG CTT CAC TTT CCA TTT CTG CCT CGT TCT
Gln Tyr Leu Asp Arg Ile Glu Val Lys Gly Lys Asp Gly Ala Arg
        705                 710                 715

TGG CAC TTT GGT GCA GTT GCG CAG CAT GTT ATC AGT GTA TTT CAG
--------------------------------------------------------- 2504
ACC GTG AAA CCA CGT CAA CGC GTC GTA CAA TAG TCA CAT AAA GTC
Trp His Phe Gly Ala Val Ala Gln His Val Ile Ser Val Phe Gln
        720                 725                 730

AAT GAA GGC ATA GAT GTG TCA CGA CTG GCA TTT ATC TGT TAT GAC
--------------------------------------------------------- 2549
TTA CTT CCG TAT CTA CAC AGT GCT GAC CGT AAA TAG ACA ATA CTG
Asn Glu Gly Ile Asp Val Ser Arg Leu Ala Phe Ile Cys Tyr Asp
        735                 740                 745

AAG TGG AAT GAG ACC CCG GCA GAA TAC AGG GAT GTG ACG GAA GAA
--------------------------------------------------------- 2594
TTC ACC TTA CTC TGG GGC CGT CTT ATG TCC CTA CAC TGC CTT CTT
Lys Trp Asn Glu Thr Pro Ala Glu Tyr Arg Asp Val Thr Glu Glu
        750                 755                 760

GAG CAT TCT GCA GGA GTT TAC CCA CTT ATA CAG ACA AAG GTT CTG
--------------------------------------------------------- 2639
CTC GTA AGA CGT CCT CAA ATG GGT GAA TAT GTC TGT TTC CAA GAC
Glu His Ser Ala Gly Val Tyr Pro Leu Ile Gln Thr Lys Val Leu
        765                 770                 775

GTA CGC GAA GCC GTC GAG GCT GGT GAA TGT TAC GGT ATC CGT TAT
--------------------------------------------------------- 2684
CAT GCG CTT CGG CAG CTC CGA CCA CTT ACA ATG CCA TAG GCA ATA
Val Arg Glu Ala Val Glu Ala Gly Glu Cys Tyr Gly Ile Arg Tyr
        780                 785                 790

GAA GAG GCT CTG ATT CTG GAA TCT GCG ATG ATG AGA CGC AGG GTT
--------------------------------------------------------- 2729
CTT CTC CGA GAC TAA GAC CTT AGA CGC TAC TAC TCT GCG TCC CAA
Glu Glu Ala Leu Ile Leu Glu Ser Ala Met Met Arg Arg Arg Val
        795                 800                 805

AAA AAG CTG GAA GAG CAA GTT TTG CAA TTA ACA GGG AAT
---------------------------------------------------     2768
TTT TTC GAC CTT CTC GTT CAA AAC GTT AAT TGT CCC TTA
Lys Lys Leu Glu Glu Gln Val Leu Gln Leu Thr Gly Asn
        810                 815                 820
```

```
TGAACCGTAA ATGGTGTGTT GTTGCGCGGT ATACTTTTCC TGAAGCAGGG
- - - - - - - - - - - - - - - - - - - - - - - - - - - - - - - - 2818
ACTTGGCATT TACCACACAA CAACGCGCCA TATGAAAAGG ACTTCGTCCC

TGTTTGCAAA TAAACGGGTT TCGTTATGTC ATTCCAACTA ACCAATGAAA
- - - - - - - - - - - - - - - - - - - - - - - - - - - - - - - - 2868
ACAAACGTTT ATTTGCCCAA AGCAATACAG TAAGGTTGAT TGGTTACTTT

CTTCAAATCA GTGGCTTAGT GTTAGTTCTC TTGCTGCGGT TATTGCAGGT
- - - - - - - - - - - - - - - - - - - - - - - - - - - - - - - - 2918
GAAGTTTAGT CACCGAATCA CAATCAAGAG AACGACGCCA ATAACGTCCA

GTCCCTCCGG AGGTTGCTTT GGGGGCTTTG GCTGGGGCGG TAATTTTTGT
- - - - - - - - - - - - - - - - - - - - - - - - - - - - - - - - 2968
CAGGGAGGCC TCCAACGAAA CCCCCGAAAC CGACCCCGCC ATTAAAAACA

TACCTCTGCG GTAGAGTATC CTATTCGTCG TCGTGTACTC TTGTCGATGC
- - - - - - - - - - - - - - - - - - - - - - - - - - - - - - - - 3018
ATGGAGACGC CATCTCATAG GATAAGCAGC AGCACATGAG AACAGCTACG

TTAGCTTTCT CTGCGGCCTT CTTTTTTATA AACCAGCAGC ATCAATTCTT
- - - - - - - - - - - - - - - - - - - - - - - - - - - - - - - - 3068
AATCGAAAGA GACGCCGGAA GAAAAAATAT TTGGTCGTCG TAGTTAAGAA

ATCGGCATAG CCAGCCTGAT C
- - - - - - - - - - - - -   3089
TAGCCGTATC GGTCGGACTA G
```

## Revendications

**1 -** Fragment d'ADN portant un gène codant pour une enzyme destinée à la fragmentation du N-acétylhéparosane de haute masse moléculaire et les systèmes d'expression permettant sa biosynthèse caractérisé en ce qu'il a 3089 pb et correspond à la séquence nucléotidique SEQ ID N° 1.

**2 -** Fragment d'ADN codant pour la séquence peptidique SEQ ID N° 2.

**3 -** Fragment d'ADN codant pour tout ou partie de la séquence peptidique SEQ ID N° 2.

**4 -** Séquence peptidique constituée ou comprenant la séquence SEQ ID N° 2.

**5 -** Gène recombinant codant pour une enzyme destinée à la fragmentation du N-acétylhéparosane porté par un fragment d'ADN correspondant à la séquence nucléotidique selon la revendication 1.

**6 -** Gène recombinant codant pour une enzyme destinée à la fragmentation du N-acétylhéparosane porté par un fragment d'ADN codant pour tout ou partie de la séquence peptidique selon la revendication 4.

**7 -** Souche portant un plasmide permettant la production d'une enzyme recombinée destinée à la fragmentation du N-acétylhéparosane de haute masse moléculaire, caractérisée en ce qu'elle est obtenue par transformation de la souche Escherichia coli RRI (K12) ou une autre souche équivalente, avec un vecteur portant un fragment d'ADN correspondant à la séquence SEQ ID N° 1.

**8 -** Souche déposée auprès de la CNCM de l'Institut Pasteur, Paris, France sous le numéro I-1352.

**9 -** Enzyme recombinée destinée à la fragmentation du N-acétylhéparosane de haute masse moléculaire issue d'un gène selon l'une quelconque des revendications 5 ou 6.

**10 -** Enzyme recombinée destinée à la fragmentation du N-acétylhéparosane de haute masse moléculaire issue d'un gène comportant une séquence d'ADN codant pour la séquence peptidique selon la revendication 4.

**11 -** La souche TP 2339 transformée par le plasmide p868,26.

**12 -** Le plasmide p868,26.

**13 -** Préparation contenant une enzyme selon l'une quelconque des revendications 9 ou 10.

**14 -** Procédé de fragmentation du N-acétylhéparosane utilisant une enzyme selon l'une quelconque des revendications 9 ou 10.

**15 -** Utilisation d'une enzyme selon l'une quelconque des revendications 9 ou 10 sous sa forme soluble pour la fragmentation du N-acétylhéparosane de haute masse moléculaire.

Office européen des brevets

# RAPPORT DE RECHERCHE EUROPEENNE

Numero de la demande
EP 94 40 1855

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | FEMS MICROBIOLOGY LETTERS, vol.16, no.1, Janvier 1983 pages 13 - 17 DHIRENDRA S. GUPTA ET AL. 'Enzymatic degradation of the capsular K5-antigen of E. coli by coliphage K5' * page 13, colonne de gauche, alinéa 1 * * page 14, colonne de gauche, alinéa 3 - colonne de droite, alinéa 1 * * page 15, colonne de gauche, alinéa 3 - colonne de droite, alinéa 1 * * page 16, colonne de droite, alinéa 2 -alinéa 3 * | | C12N15/56 C12N15/60 C12N15/70 C12N9/24 C12N9/88 C12N1/21 //(C12N1/21, C12R1:19) |
| D,A | EP-A-0 489 647 (ELF SANOFI) * page 5, ligne 11 - ligne 22 * * page 5, ligne 45 - page 6, ligne 42 * | | |
| | | | DOMAINES TECHNIQUES RECHERCHES (Int.Cl.6) |
| | | | C12N C12R |

Le présent rapport a été établi pour toutes les revendications

| Lieu de la recherche | Date d'achèvement de la recherche | Examinateur |
|---|---|---|
| LA HAYE | 12 Décembre 1994 | MONTERO LOPEZ B. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)